# EUROPEAN PATENT APPLICATION

(11) **EP 4 257 132 A1**
(43) Date of publication of application: **11.10.2023**
(21) Application number: 22167466.6
(22) Date of filing: 08.04.2022
(51) Int. Cl.: A61K 31/506, A61K 38/17, A61K 45/06, A61K 39/00, A61P 35/00

(54) **SIK3 INHIBITORS FOR TREATING DISEASES RESISTANT TO DEATH RECEPTOR SIGNALLING**

(71) Applicant: iOmx Therapeutics AG, 82152 Martinsried (DE)
(72) Inventor: MASER, Petra, 82152 Martinsried (DE); BISSINGER, Stefan, 82152 Martinsried (DE)
(74) Representative: Kuttenkeuler, David

(57) **Abstract**

The invention is based on the surprising finding that SIK3 is associated with resistance against anti-tumour immune responses that are dependent on tumor necrosis factor related apoptosis-inducing ligand (TRAIL). In particular, the invention provides compounds for use in treating and methods for treating proliferative diseases using inhibitors of SIK3, especially nucleic acid or small molecule inhibitors of SIK3 for treating proliferative disorders that benefit from TRAIL or TRAIL receptor induced apoptosis. In certain aspects the invention therefore provides SIK3 inhibitors for use in the treatment of a disease characterized by a resistance to TRAIL, wherein the SIK3 inhibtior is used as a TRAIL sensitizer. As such, the inventon therefore provides specific treatment options in tumor diseases that are characterized by a certain concentration of intratumoural or plasma concentration of TRAIL. Also provided are methods of sensitising cells involved with a proliferative disorder against the cytotoxic effect of TRAIL and its receptors, and/or to kill such cells and/or methods for treating proliferative diseases, using a SIK3 inhibitor together with TRAIL, TRAIL ligands or agonists of TRAIL signalling pathways.

## Description

The invention is based on the surprising finding that SIK3 is associated with resistance against anti-tumour immune responses that are dependent on tumor necrosis factor related apoptosis-inducing ligand (TRAIL). In particular, the invention provides compounds for use in treating and methods for treating proliferative diseases using inhibitors of SIK3, especially nucleic acid or small molecule inhibitors of SIK3 for treating proliferative disorders that benefit from TRAIL or TRAIL receptor induced apoptosis. In certain aspects the invention therefore provides SIK3 inhibitors for use in the treatment of a disease characterized by a resistance to TRAIL, wherein the SIK3 inhibtior is used as a TRAIL sensitizer. As such, the inventon therefore provides specific treatment options in tumor diseases that are characterized by a certain concentration of intratumoural or plasma concentration of TRAIL. Also provided are methods of sensitising cells involved with a proliferative disorder against the cytotoxic effect of TRAIL and its receptors, and/or to kill such cells and/or methods for treating proliferative diseases, using a SIK3 inhibitor together with TRAIL, TRAIL ligands or agonists of TRAIL signalling pathways.

In the treatment of cancer there are a number of approaches by which therapies may lead to the elimination of tumour cells, including those that involve or exploit one or more components of the immune system, either directly or indirectly. One of the limitations associated with such therapies is that cancerous cells often exploit immune-checkpoints to evade a patient's immune system, such by preventing immune-recognition or down-regulating a tumour-specific cytotoxic T cell (CTL) response, thereby generating resistance against an immune response (Rabinovich et al 2007, Annu Rev Immunol 25:267; Zitvogel et al 2006, Nat Rev Immunol 6:715). Under normal conditions, such immune-regulatory checkpoints are crucial for the maintenance of self-tolerance under physiological conditions but there is an increasing recognition of the important role that they can also play in cancer (Hanahan and Weinberg 2011, Cell; 144:646); cancerous cells can take over these mechanisms to evade and suppress the immune system in order to develop into a tumour (Drake et al 2006, Adv Immunol 90:51).

Current state of the art cancer therapies include blockade of those few immune-regulatory checkpoints presently known and for which their mechanism of action is understood. For example, blocking antibodies against surface-expressed immune-regulatory proteins, such as CTLA4 and PD-L1 (Chambers et al 2001, Annu Rev Immunol 19:565; Blank et al 2004, Cancer Res 64:1140), can boost anti-tumour immunity and have shown clinical success against many cancer types (Page et al 2014, Annu Rev Med 65:185). However, a large proportion of cancer patients does not respond to such checkpoint blockage therapy (Bu et al 2016, Trends Mol Med 22:448; Hugo et al 2016, Cell 165:35; Topalian et al 2012, New Engl J Med 366:2443), indicating that other immune-checkpoint pathways may be active. Indeed, synergistic cooperation between several immune-regulatory pathways maintains immune tolerance against tumours, which might explain why blocking only one immune-regulatory checkpoint node can still result in tumour escape (Woo et al 2012, Cancer Res 72:917; Berrien-Elliott et al 2013, Cancer Res 73:605). However, little is known about the molecular factors that are central to the mechanism of action of such immune-regulatory pathways. Indeed, successful cancer immunotherapy requires a systematic delineation of the entire immune-regulatory circuit - the 'immune modulatome '- expressed by tumours. Therefore, today, there is still an unmet need for identifying further molecular targets that may serve as immune-regulatory checkpoints and in particular an unmet need for means and methods to modulate, detect and otherwise utilise such possible checkpoint targets, such as in medicine, diagnosis and research.

Salt-inducible kinases (SIKs) constitute a serine tyrosine kinase subfamily, belonging to the adenosine monophosphate-activated kinase (AMPK) family. Three members (SIK1, -2, and -3) have been identified so far. Amino acid homology of SIK1 with SIK2 and SIK3 is 78% and 68%, respectively, in the kinase domain. The cloning of SIK1

(also known as SIK and SNF1LK), abundantly expressed in the adrenal glands of high-salt, diet-fed rats, led to subsequent cloning of SIK2 (also known as QIK, KIAA0781 and SNF1LK2), mainly expressed in adipose tissues and the rather ubiquitous SIK3 (also known as QSK, KIAA0999 or L19) (Katoh et al. 2004, Mol. Cell. Endocrinol. 217:109). The three SIKs have a similar structure, with an N-terminal kinase domain (catalytic domain), a middle ubiquitin-associated domain (believed important for phosphorylation by LKB1) and a long C-terminal sequence (believed to be a site for further phosphorylation by PKA). However, there are very diverse roles implicated for the various SIKs. For example, various SIKs have been implicated in biological processes as diverse as osteocyte response to parathyroid hormone (Wein et al. 2016, Nature Commun. 7:13176) to induction of SIK1 by gastrin and inhibition of migration of gastric adenocarcinoma cells (Selvik et al. 2014, PLoS ONE 9:e112485). Other potential roles of salt-inducible kinases (in particular SIK3) are described in WO2018/193084A1 (to the present applicant, and published 25-Oct-2018) furthermore that SIK3 is a gene involved in tumour cell resistance to cell-mediated immune responses, in particular tumour cell resistance to TNF. Recently, SIKs (particularly SIK3) have been demonstrated to also regulate TGFbeta-mediated transcriptional activity and apoptosis, with Hutchinson et al (2010, Cell Death and Disease 11:49) showing that SIK3 expression or activity results in resistance to TGFbeta-mediated apoptosis.

In particular, as well as playing a role in various inflammatory responses (Clark et al 2014; Sundberg et al 2016) and oncology - especially the sensitisation of tumour cells to immune responses (WO2018/193084A1) - it has been known since 2011 that inhibition of SIK2 promotes melanogenesis in B16F10 melanoma cells (Kumagai et al 2011, PLoS ONE 6(10): e26148). It was subsequently described that the pigmentation pathway including in human skin explants can be efficaciously induced by (topical) treatment with SIK inhibitors, including those structurally related to YKL-05-099 (Mujahid et al 2017, Cell Reports 19:2177). Indeed, using such results, it has subsequently been sought to claim methods of increasing (the appearance of) skin pigmentation in a subject by administering topically to the subject skin an effective account of a SIK inhibitor (WO2018/160774), including using kinase inhibitors previously known to be SIK inhibitors (WO2016/023014). In particular, the prior art excluded any role of TRAIL mediated apoptosis in the SIK3 pathway.

It is thus an object of the invention to fully understand the clinical relevance of SIk3 mediated inhibition in the context of immune oncology and to develop and provide novel therapeutic avenues for treating cancer.

### SUMMARY OF THE INVENTION

Generally, and by way of brief description, the main aspects of the present invention can be summarised as follows:

In **a first aspect,** the present invention provides a **SIK3 inhibitor for use in a treatment of a proliferative disorder** in a subject, wherein the treatment involves: (i) inhibiting SIK3; and thereby sensitising cells involved with the proliferative disorder to TRAIL mediated apoptosis and/or a cell-mediated immune response involving TRAIL, and/or (ii) inhibiting SIK3 thereby re-sensitizing a TRAIL resistant cell involved with the proliferative disorder to TRAIL mediated apoptosis.

In **a related first aspect,** the present invention provides a **SIK3 inhibitor for use in a treatment of a proliferative disorder** in a subject, wherein the treatment involves inhibiting SIK3; and thereby re-sensitising cells involved with the proliferative disorder to TRAIL mediated apoptosis, in particular wherein the proliferative disorder is characterized by a resistance to TRAIL.

In **a second aspect,** the present application provides **a SIK3 inhibitor for use in a treatment of a proliferative disorder** in a subject, the treatment comprising exposing cells involved with the proliferative disorder in the subject to: (i) TRAIL and/or an agonist of a TRAIL receptor (DR4 or DR5) signalling; and (ii) the SIK3 inhibitor wherein: (i) TRAIL and/or an agonist of a TRAIL receptor (DR4 or DR5) signalling is administered to the subject; (ii) an agent that is capable of inducing or induces the exposure of the cells involved with the proliferative disorder to TRAIL and/or an agonist of a TRAIL receptor (DR4 or DR5) signalling, is administered to the subject; or (iii) the exposure of the cells involved with the proliferative disorder to TRAIL is induced by a pharmaceutical, therapeutic or other procedure that increases the amount of TRAIL in the plasma of the subject and/or in the environment of such cells; optionally, wherein the treatment comprises that the SIK3 inhibitor is administered to the subject in a therapeutically effective amount effective to inhibit SIK3, in particular SIK3 in the cells involved with the proliferative disorder.

In **a third aspect,** the present invention pertains to **a substance or composition for use in a treatment of a cancer or tumour** in a subject, the treatment comprising exposing cells involved with the proliferative disorder in the subject to: (i) TRAIL and/or an agonist of a TRAIL receptor (DR4 or DR5) signalling; and (ii) a SIK3 inhibitor; wherein exposure of the cells involved with the proliferative disorder to TRAIL is induced by administration of the ligand, wherein the substance is a ligand that binds to an immune checkpoint molecule, or wherein the composition comprises a ligand that binds to an immune checkpoint molecule.

In **a fourth aspect,** the present application provides **a SIK3 inhibitor for use in a treatment of a proliferative disorder** in a subject, the treatment comprising exposing cells involved with the proliferative disorder in the subject to: the SIK3 inhibitor, wherein: in the cells involved with the proliferative disorder is characterized by a reduced or absent sensitivity to TRAIL induced signalling, in particular TRAIL induced cell death (such as apoptosis).

In **a related aspect,** the present invention provides a pharmaceutical composition comprising TRAIL or an agonist of TRAIL signalling and an inhibitor of Salt Inducible Kinase 3 (SIK3 inhibitor), together with a pharmaceutical acceptable carrier and/or excipient.

In a related aspect, the present invention

Yet **further aspects** of the invention are **disclosed** herein.

### BRIEF DESCRIPTION OF THE FIGURES

The figures show:

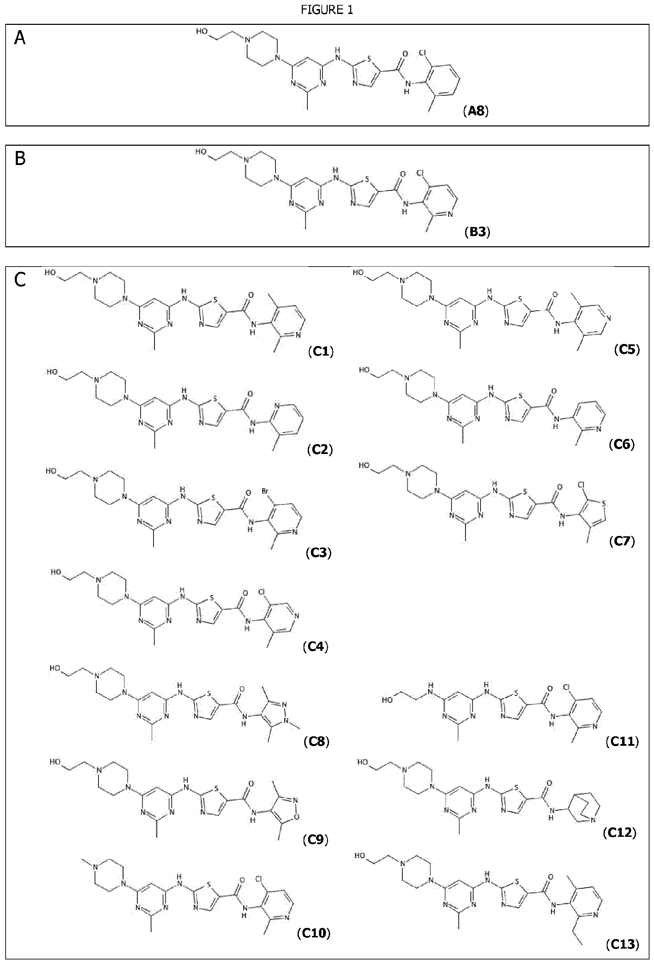

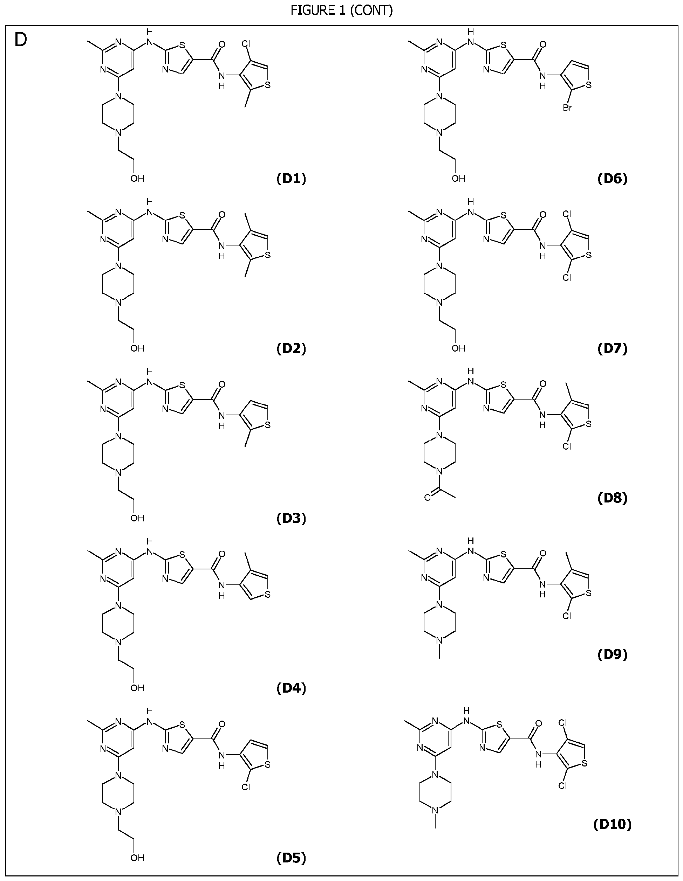

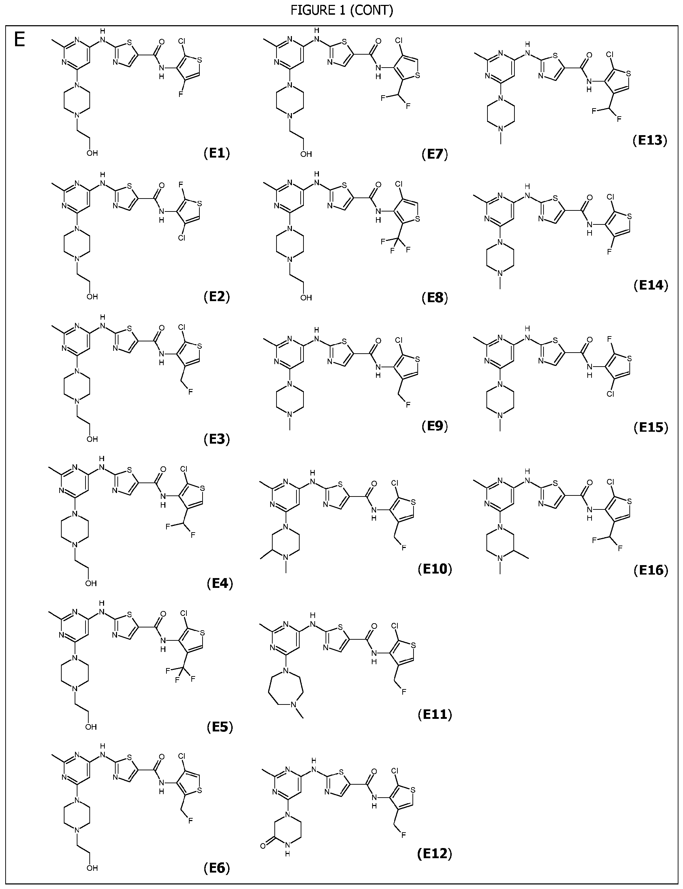

**Figure 1****:** depicts the chemical structures of: (A) dasatinib (compound A8), N-(2-chloro-6-methylphenyl)-2-((6-(4-(2-hydroxyethyl)piperazin-1-yl)-2-methylpyrimidin-4-yl)amino)thiazole-5-carboxamide; (B) the kinase inhibitor B3, N-(4-chloro-2-methylpyridin-3-yl)-2-((6-(4-(2-hydroxyethyl)piperazin-1-yl)-2-methylpyrimidin-4-yl)amino)thiazole-5-carboxamide; (C) certain other kinase inhibitors of formula (Ib)/(Ic) C1 to C13; (D) certain further kinase inhibitors of formula (Ib)/(Ic) D1 to D10; and (E) certain kinase inhibitors of formula (Ia) E1 to E16.

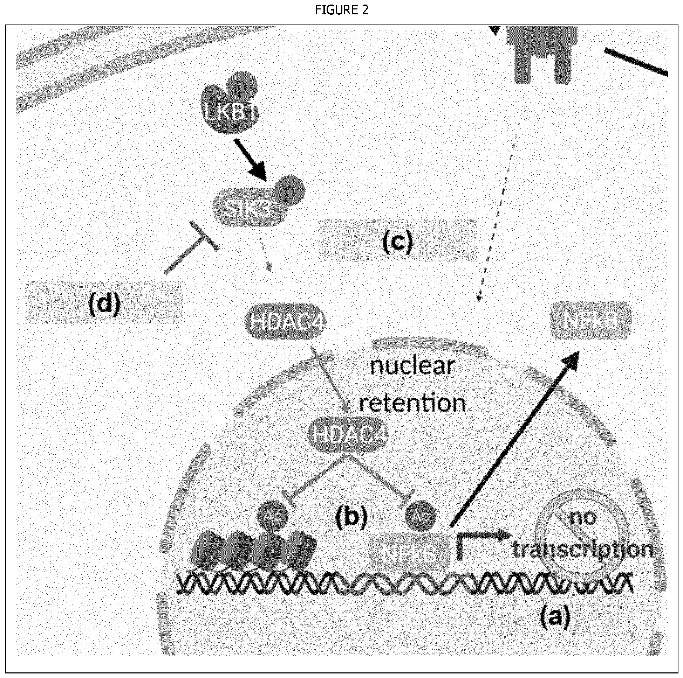

**Figure 2****:** depicts a schematic of SIK3-mediated control of the expression of survival/maintenance genes (a) by nuclear factor κB (NFκB) transcriptional regulation (b)), NFκB activity is controlled by presence in the nucleus of HDAC4 acting, as a repressive co-factor, whose retention in the cytoplasm is brought about by its phosphorylation by SIK3. Nuclear entry and presence (c) of un-phosphorylated HDAC4 (and hence reduction of expression of tumour survival/maintenance genes (a) by inhibition of the transcription-factor activity of NFκB) can be brought about by inhibition of SIK3 by a compound disclosed herein (d) such as compound E9, and thereby apoptosis is enhanced.

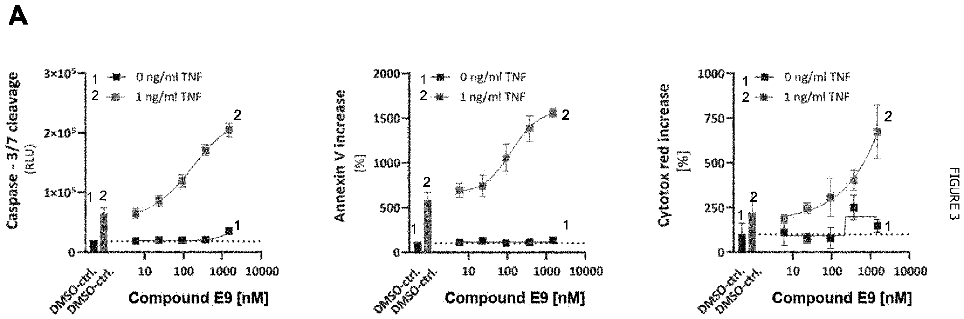

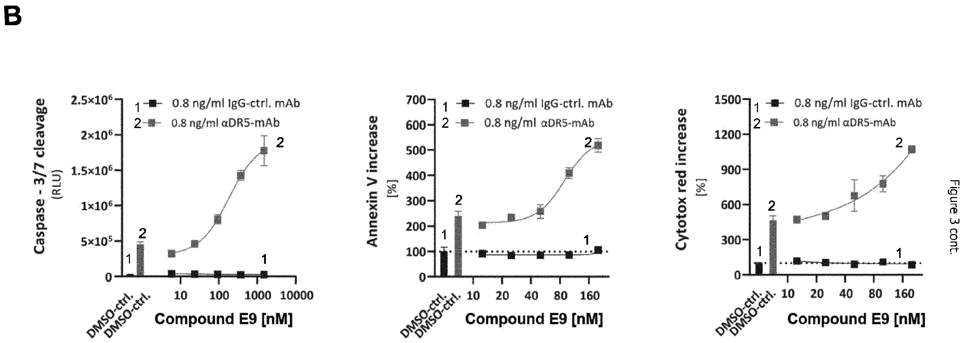

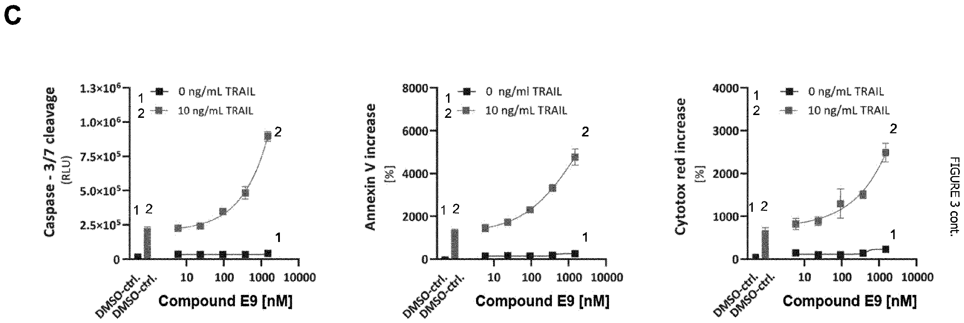

**Figure 3****:** Murine colorectal carcinoma cell line MC38 (A), murine triple negative breast cancer cell line EMT6 (B) and human melanoma tumor cell line A375 (C) were treated with 1 ng/ml TNF, 0.8 ng/ml murine death receptor 5 targeting antibody (clone MD5-1), 10 ng/ml complexed TRAIL or appropriate controls in combination with SIK3 inhibitor E9 and an associated DMSO-control. The onset of tumor cell death was monitored 8 hours after treatment initiation by cleavage of effector caspases 3/7, while the increase in extracellular phosphatidylserine expression was analyzed by annexin V and cell membrane disruption by cytotox red staining 24 hours after therapy initiation.

### DETAILS OF THE PRESENT INVENTION

The present invention, and particular non-limiting aspects and/or embodiments thereof, can be described in more detail as follows.

Although the present invention may be further described in more detail, it is to be understood that this invention is not limited to the particular methodologies, protocols and reagents described herein as these may vary. It is also to be understood that the terminology used herein is for the purpose of describing particular embodiments only, and is not intended to limit the scope of the present invention which will be limited only by what is described, defined or otherwise disclosed herein, in particular in any itemised embodiments or the appended claims.

Herein, certain elements of the present invention are described in more detail. These elements are listed with specific embodiments, however, it should be understood that they may be combined in any manner and in any number to create additional embodiments. The variously described examples and preferred embodiments should not be construed to limit the present invention to only the explicitly described embodiments. This description of this application should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise. For example, if in one embodiment of the compound of the invention L is a bond and in another embodiment of the compound of the invention R³ is H, then in a preferred embodiment of the compound of the invention, L is a bond and R³ is H, or if in one embodiment of the use of a compound of the invention the subject is an adult human and in another embodiment of the use of a compound of the invention the proliferative disorder is prostate cancer, then in a preferred embodiment of the use of a compound of the invention, the subject is an adult human and the proliferative disorder is prostate cancer.

### General definitions

Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", H.G.W. Leuenberger, B. Nagel, and H. Kölbl, Eds., Helvetica Chimica Acta, CH-4010 Basel, Switzerland, (1995).

The practice of the present invention will employ, unless otherwise indicated, conventional methods of chemistry, biochemistry, and recombinant DNA techniques which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps. The term "consisting essentially of" means excluding other members, integers or steps of any essential significance or group of members, integers or steps of any essential significance. For example, a pharmaceutical composition consisting essentially of the members/components as defined herein (such as a compound as defined in any of the aspects of the invention and optionally one additional therapeutic agent) would exclude further therapeutic agents (besides the compound as defined in any of the aspects of the invention and the optional one additional therapeutic agent) but would not exclude contaminants (e.g., those from the isolation and purification method) in trace amounts (e.g., the amount of the contaminant (preferably the amount of all contaminants present in the composition) is less than 5% by weight, such as less than 4% by weight, 3% by weight, 2% by weight, 1% by weight, 0.5% by weight, 0.4% by weight, 0.3% by weight, 0.2% by weight, 0.1% by weight, 0.05% by weight, with respect to the total composition) and/or pharmaceutically acceptable excipients (such as carriers, e.g., phosphate buffered saline, preservatives, and the like). The term "consisting of" means excluding all other members, integers or steps of significance or group of members, integers or steps of significance. For example, a pharmaceutical composition consisting of the members/components as defined herein (such as a compound as defined in any of the aspects of the invention, one excipient, and optionally one additional therapeutic agent) would exclude any other compound (including a second or further excipient) in an amount of more than 2% by weight (such as any other compound in an amount of more than 1% by weight, more than 0.5% by weight, more than 0.4% by weight, more than 0.3% by weight, more than 0.2% by weight, more than 0.1% by weight, more than 0.09% by weight, more than 0.08% by weight, more than 0.07% by weight, more than 0.06% by weight, more than 0.05% by weight, more than 0.04% by weight, more than 0.03% by weight, more than 0.02% by weight, more than 0.01% by weight) with respect to the total composition. The term "comprising" encompasses the term "consisting essentially of' which, in turn, encompasses the term "consisting of'. Thus, at each occurrence in the present application, the term "comprising" may be replaced with the term "consisting essentially of' or "consisting of'. Likewise, at each occurrence in the present application, the term "consisting essentially of' may be replaced with the term "consisting of".

Where used herein, "and/or" is to be taken as specific disclosure of each of the two specified features or components with or without the other. For example, "X and/or Y" is to be taken as specific disclosure of each of (i) X, (ii) Y, and (iii) X and Y, just as if each is set out individually herein.

In the context of the present invention, the terms "about" and "approximately" are used interchangeably and denote an interval of accuracy that the person of ordinary skill will understand to still ensure the technical effect of the feature in question. The term typically indicates deviation from the indicated numerical value by ±5%, ±4%, ±3%, ±2%, ±1%, ±0.9%, ±0.8%, ±0.7%, ±0.6%, ±0.5%, ±0.4%, ±0.3%, ±0.2%, ±0.1%, ±0.05%, and for example ±0.01%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect.

The terms "a", "an" and "the" and similar references used in the context of describing the invention (especially in the context of the claims) are to be construed to cover both the singular and the plural, unless otherwise indicated herein or clearly contradicted by the context.

Recitation of ranges of values herein is merely intended to serve as a shorthand method of referring individually to each separate value falling within the range. Unless otherwise indicated herein, each individual value is incorporated into the specification as if it were individually recited herein.

All methods described herein can be performed in any suitable order unless otherwise indicated herein or otherwise clearly contradicted by the context.

The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention.

Several documents are cited throughout the text of this specification. Each of the documents cited herein (including all patents, patent applications, scientific publications, manufacturer's specifications, instructions, etc.), whether *supra* or *infra,* are hereby incorporated by reference in their entirety. Nothing herein is to be construed as an admission that the invention is not entitled to antedate such disclosure by virtue of prior invention.

The terms "of the [present] invention", "in accordance with the [present] invention", "according to the [present] invention" and the like, as used herein are intended to refer to all aspects and embodiments of the invention described and/or claimed herein.

It is to be understood that the application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above or below are not limited to any particular aspect or embodiment of the invention and apply equally to all aspects and embodiments that are described.

"Polymorphism" as referred to herein means that a solid material (such as a compound) is able to exist in more than one form or crystalline structure, i.e., "polymorphic modifications" or "polymorphic forms". The terms "polymorphic modifications", "polymorphic forms", and "polymorphs" are used interchangeable in the present invention. According to the present invention, these "polymorphic modifications" include crystalline forms, amorphous forms, solvates, and hydrates. Mainly, the reason for the existence of different polymorphic forms lies in the use of different conditions during the crystallization process, such as the following:
- solvent effects (the packing of crystal may be different in polar and nonpolar solvents);
- certain impurities inhibiting growth pattern and favor the growth of a metastable polymorphs;
- the level of supersaturation from which material is crystallized (in which generally the higher the concentration above the solubility, the more likelihood of metastable formation);
- temperature at which crystallization is carried out;
- geometry of covalent bonds (differences leading to conformational polymorphism);
- change in stirring conditions.

Polymorphic forms may have different chemical, physical, and/or pharmacological properties, including but not limited to, melting point, X-ray crystal and diffraction pattern, chemical reactivity, solubility, dissolution rate, vapor pressure, density, hygroscopicity, flowability, stability, compactability, and bioavailability. Polymorphic forms may spontaneously convert from a metastable form (unstable form) to the stable form at a particular temperature. According to Ostwald's rule, in general it is not the most stable but the least stable polymorph that crystallizes first. Thus, quality, efficacy, safety, processability and/or manufacture of a chemical compound, such as a compound of the present invention, can be affected by polymorphism. Often, the most stable polymorph of a compound (such as a compound of the present invention) is chosen due to the minimal potential for conversion to another polymorph. However, a polymorphic form which is not the most stable polymorphic form may be chosen due to reasons other than stability, e.g. solubility, dissolution rate, and/or bioavailability.

The term "crystalline form" of a material as used herein means that the smallest components (i.e., atoms, molecule or ions) of said material form crystal structures. A "crystal structure" as referred to herein means a unique three-dimensional arrangement of atoms or molecules in a crystalline liquid or solid and is characterized by a pattern, a set of atoms arranged in a particular manner, and a lattice exhibiting long-range order and symmetry. A lattice is an array of points repeating periodically in three dimensions and patterns are located upon the points of a lattice. The subunit of the lattice is the unit cell. The lattice parameters are the lengths of the edges of a unit cell and the angles between them. The symmetry properties of the crystal are embodied in its space group. In order to describe a crystal structure the following parameters are required: chemical formula, lattice parameters, space group, the coordinates of the atoms and occupation number of the point positions.

The term "amorphous form" of a material as used herein means that the smallest components (i.e., atoms, molecule or ions) of said material are not arranged in a lattice but are arranged randomly. Thus, unlike crystals in which a short-range order (constant distances to the next neighbor atoms) and a long-range order (periodical repetition of a basic lattice) exist, only a short-range order exists in an amorphous form.

The term "complex of a compound" as used herein refers to a compound of higher order which is generated by association of the compound with one or more other molecules. Exemplary complexes of a compound include, but are not limited to, solvates, clusters, and chelates of said compound.

The term "solvate" as used herein refers to an addition complex of a dissolved material in a solvent (such as an organic solvent (e.g., an aliphatic alcohol (such as methanol, ethanol, n-propanol, isopropanol), acetone, acetonitrile, ether, and the like), water or a mixture of two or more of these liquids), wherein the addition complex exists in the form of a crystal or mixed crystal. The amount of solvent contained in the addition complex may be stoichiometric or non-stoichiometric. A "hydrate" is a solvate wherein the solvent is water.

In isotopically labeled compounds one or more atoms are replaced by a corresponding atom having the same number of protons but differing in the number of neutrons. For example, a hydrogen atom may be replaced by a deuterium atom. Exemplary isotopes which can be used in the compounds of the present invention include deuterium, ¹¹C, ¹³C, ¹⁴C, ¹⁵N, ¹⁸F, ³²P, ³²S, ³⁵S, ³⁶Cl, and ¹²⁵I.

The expression "amino protecting group" as used herein preferably refers to any group by which an amino group contained in a compound can be transferred into a less reactive (i.e., protected) amino group. Preferably, amino protecting groups can be incorporated into the corresponding compound under mild conditions, in a chemoselective and/or regioselective manner, and/or in good yields. Furthermore, the amino protecting groups should be stable under the conditions to which the protected compound is to be subjected (e.g., the conditions of the desired reaction and/or purification conditions). Preferably, the amino protecting groups should minimize the risk of racemization of a stereogenic center, when present in the compound. In one embodiment, the amino protecting groups should be removable from the protected compound under mild conditions and in a selective manner such that the deprotected compound is obtained in high yields. Exemplary amino protecting groups include tert-butyloxycarbonyl (BOC), 9-fluorenylmethoxycarbonyl (FMOC), benzyloxycarbonyl (Cbz), p-methoxybenzylcarbonyl (MOZ), acetyl (Ac), trifluoroacetyl, benzoyl (Bz), benzyl (Bn), p-methoxybenzyl (PMB), 3,4-dimethoxyphenyl (DMPM), p-methoxyphenyl (PMP), 2,2,2-trichloroethoxycarbonyl (Troc), triphenylmethyl (trityl; Tr), toluenesulfonyl (tosyl; Ts), *para-*bromophenylsulfonyl (brosyl), 4-nitrobenzenesulfonyl (nosyl), and 2-nitrophenylsulfenyl (Nps).

The term "half-life" relates to the period of time which is needed to eliminate half of the activity, amount, or number of molecules. In the context of the present invention, the half-life of a compound disclosed herein (eg a compound of formula (Ia), (Ib) or (Ic)) is indicative for the stability of said compound.

The terms "subject", "patient", "individual", or "animal" relate to multicellular animals, such as vertebrates. For example, vertebrates in the context of the present invention are mammals, birds (e.g., poultry), reptiles, amphibians, bony fishes, and cartilaginous fishes, in particular domesticated animals of any of the foregoing as well as animals (in particular vertebrates) in captivity such as animals (in particular vertebrates) of zoos. Mammals in the context of the present invention include, but are not limited to, humans, non-human primates, domesticated mammals, such as dogs, cats, sheep, cattle, goats, pigs, horses etc., laboratory mammals such as mice, rats, rabbits, guinea pigs, etc. as well as mammals in captivity such as mammals of zoos. The term "animal" as used herein also includes humans. Particular non-limiting examples of birds include domesticated poultry, and include birds such as chickens, turkeys, ducks, geese, guinea fowl, pigeons, pheasants etc.; while particular non-limiting examples of bony or cartilaginous fish include those suitable for cultivation by aquiculture, and include bony fish such as salmon, trout, perch, carp, cat-fish, etc.

### Sensitisation to TRAIL or to immune responses involving TRAIL

The compounds of the invention can sensitise cells involved with a proliferative disorder to a TRAIL induced apoptosis and/or to a cell-mediated immune response that involves TRAIL as an effector molecule.

Accordingly, in one embodiment, a treatment comprising administering a compound (or a pharmaceutical composition) of the invention to the subject involves (eg, is mediated, is or supported) sensitising cells involved with the proliferative disorder (in the subject) to a cell-mediated immune response involving, and/or mediated by TRAIL or one of its receptors.

In an alternative embodiment, a treatment comprising administering a compound (or a pharmaceutical composition) of the invention to the subject involves (eg, is mediated, is or supported by) inhibiting a kinase involved in resistance to a cell-mediated immune response, such as inhibiting SIK3 (in the subject). As such a compound in this case may be a SIK3 inhibitor as defined herein.

In a related embodiment, a treatment comprising administering a compound (or a pharmaceutical composition) of the invention to the subject involves (eg, is mediated, is or supported by) inhibiting a kinase involved in resistance to a cell-mediated immune response involving TRAIL, such as inhibiting SIK3, and (for example, thereby) sensitising cells involved with the proliferative disorder (in the subject) to TRAIL mediated apoptosis and/or a cell-mediated immune response involving TRAIL.

In **a further aspect,** and as may be further described, defined, claimed or otherwise disclosed herein, the invention relates to **a method for the sensitisation of cells** involved with a proliferative disorder to TRAIL mediated apoptosis and/or to a cell-mediated immune response involving TRAIL in the treatment of the proliferative disorder in a subject, the method comprising administering a compound (or a pharmaceutical composition) of the invention to the subject; and in another further aspect, and as may be further described, defined, claimed or otherwise disclosed herein, the invention relates to a method for the inhibition of a kinase (in the subject) involved in resistance to a cell-mediated immune response, such as inhibiting, in the treatment of a proliferative disorder in a subject, the method comprising administering a compound (or a pharmaceutical composition) of the invention to the subject.

In a **related further aspect,** and as may be further described, defined, claimed or otherwise disclosed herein, the invention relates to **a compound (or a pharmaceutical composition)** of the invention **for use as a medicament** for: (i) sensitising cells involved with a proliferative disorder (in the subject) to TRAIL induced apoptosis and/or a cell-mediated immune response involving TRAIL; and/or (ii) inhibiting a kinase involved in resistance to a cell-mediated immune response, such as inhibiting SIK (in the subject).

In yet **a related further aspect,** and as may be further described, defined, claimed or otherwise disclosed herein, the invention relates to **a compound (or a pharmaceutical composition)** of the invention **for use as a medicament** (eg an immuno-oncology medicament) sensitising cells involved with a proliferative disorder (such as a tumour or cancer) to TRAIL induced apoptosis and/or to a cell-mediated immune response involving TRAIL, for example sensitising cells involved with a proliferative disorder (in the subject) to killing (cell-death) that may be induced by the cell-mediated immune response involving TRAIL, for example as an effector molecule. An "immune-oncology" medicament is one that would be recognised by the person of ordinary skill, and includes a medicament that is intended to (eg, specifically designed to) enhance one or more components of the immune system of an organism (such as a human) towards cancerous or tumourous cells present in such organism. An immune-oncology medicament may be one (eg an antibody) that binds to an extrinsic immune (inhibitory) checkpoint molecule (such as one described elsewhere herein) and that (eg directly) suppresses T cell function against the cancerous or tumourous cells, or an immune-oncology medicament may be one that inhibits an immune regulator (such as SIK3, as in the present invention) that is intrinsic to the cancerous or tumourous cells where such intrinsic immune regulator does not actively (eg directly) suppress T cells but rather protects the tumour or cancer cells from an immune response via a resistance mechanism.

In **a related first aspect,** the present invention provides a **SIK3 inhibitor for use in a treatment of a proliferative disorder** in a subject, wherein the treatment involves inhibiting SIK3; and thereby re-sensitising cells involved with the proliferative disorder to TRAIL mediated apoptosis, in particular wherein the proliferative disorder is characterized by a resistance to TRAIL.

In **a related aspect,** the present application provides **a SIK3 inhibitor for use in a treatment of a proliferative disorder** in a subject, the treatment comprising exposing cells involved with the proliferative disorder in the subject to: the SIK3 inhibitor, wherein: in the cells involved with the proliferative disorder is characterized by a reduced or absent sensitivity to TRAIL induced signalling, in particular TRAIL induced cell death (such as apoptosis).

A proliferative disorder is characterized by a reduced or absent sensitivity to TRAIL induced signalling, in particular TRAIL induced cell death (such as apoptosis), if cells involved with the proliferative disorder, such as tumour cells, when contacted with a SIK3 inhibitor are more sensititve to TRAIL induced killing, compared to a cell not contacted with the SIK3 inhibitor.

In particular embodiments of such aspects, the cells involved with a proliferative disorder (in the subject) may be sensitised to killing (cell-death) by (such as induced by) the cell-mediated immune response involving TRAIL.

"Salt-inducible kinase 3" or "SIK3" (synonyms QSK and KIAA0999) is a member of a subfamily of serine/threonine protein kinases including SIK1, SIK2, and SIK3 that belong to an AMP-activated protein kinase (AMPK) family. A SIK3 protein in context of the invention is, typically, a protein kinase. Pertinent information on the human SIK3 protein is accessible on UniProt: Q9Y2K2 (Entry version 138 of 15-Mar-2017) and a SIK3 protein in context of the invention has, preferably, an amino acid sequence shown in SIK3, Entry version 138 of 15-Mar-2017 or Entry version 144 of 28-Mar-2018, which sequences are incorporated herein by reference. SIK3 is a cytoplasmatic protein with serine/threonine kinase activity which is regulated through phosphorylation of a conserved threonine residue (position 163) in the T-loop of the kinase domain by the LKB1 complex; a phosphorylation which is reported as essential for catalytic activity of SIK3 (Lizcano, J. M. et al.; EMBO J. 23, 833-843 (2004)). For the purposes of the herein disclosed invention the term "phosphorylated SIK3" shall denote a SIK3 protein that is phosphorylated substantially as SIK3 protein can be (eg is) phosphorylated by LKB1, wherein preferably such phosphorylated SIK3 comprising a phosphorthreonine at amino acid position 163. A phosphorylated SIK3 in context of the invention is an SIK3 protein that is activated in its cell-biological context. At least four protein isoforms (SIK3-001 to SIK3-004) generated by alternative splicing of the SIK3 gene product are known. The human SIK3 gene is located at chromosomal position 11q23.3 (HGNC gene Symbol Acc: HGNC:29165), and is conserved in many species such as in chimpanzee, Rhesus monkey, dog, cow, mouse, rat, chicken, zebrafish, and frog. The term SIK3 in some embodiments of the invention may also pertain to variants of the human SIK3 protein having an amino acid sequence that is substantially identical to, or of at least 80%, preferably 85%, more preferably 90, 95, 96, 97, 98, 99, or 100% sequence identity to, the amino acid sequence of SIK3 as described above, as determined using, e.g., the "Blast 2 sequences" algorithm described by Tatusova & Madden 1999 (FEMS Microbiol Lett 174: 247-250), and which (preferably) retain biological activity identical or substantially identical to the respective reference SIK3 (eg to phosphorylate one or more class II (eg IIa) HDACs, such as HDAC4). Preferred variants of SIK3 protein comprise sequence variants thereof due to sequence polymorphism between and within populations of the respective species, as well as mutations compared to the wild-type sequence of SIK3 which are located in or in close proximity to the activity loop or activation loop (T-loop) of SIK3. A preferred variant of SIK3 protein is a SIK3 T163 mutation, such as a mutation affecting the activation of SIK3. In preferred embodiments a SIK3 protein of the invention is not a SIK1 (synonyms: SIK and SNF1LK) protein and/or is not a SIK2 (synonyms: QIK, KIAA0781 and SNF1LK2) protein. The amino acid sequence of human SIK1 (UniProt: P57059; entry version 168 of 15-Mar-2017) and human SIK2 (UniProt: Q9H0K1; entry version 153 of 15-Mar-2017) are incorporated herein by reference. The term SIK3 can mean, as applicable to the context (if not more specifically indicated), a SIK3 protein (such as one described above) or an mRNA molecule encoding such a SIK3 protein. The analogous meaning with respect of "SIK1" and "SIK2" is to be understood.

A compound being an "inhibitor of SIK3" (or "SIK3 inhibitor") is any moiety that inhibits SIK3, which can mean inhibition of the activity of SIK3, especially of protein of SIK3, and in particular of phosphorylated SIK3. A SIK3 inhibitor may impair (eg, induces a decrease or reduction in) the efficiency, effectiveness, amount or rate of one or more activities of SIK3, such as one or more of those activities described herein, for example, the activity of SIK3 to phosphorylate class II (eg IIa) HDACs (eg HDAC4) and/or to sensitise a cell involved with a proliferative disorder to a cell-mediated immune response.

Such a SIK3 inhibiting moiety can act directly, for example, by binding to SIK3 and decreasing the amount or rate of one or more of the properties of SIK3 such as its function, in particular its ability to act as a kinase (eg to phosphorylate HDAC4), for example by reducing the activity of phosphorylated SIK3 in the cell.

Compounds being SIK3 inhibitors are described elsewhere herein, including those as may be characterised by the applicable functional and/or structural features set out herein.

In preferred embodiments, a "subject", in particular, is also meant to include all mammals, including without limitation humans, but also non-human primates such as cynomolgus monkeys. It also includes dogs, cats, horses, sheep, goats, cows, rabbits, pigs and rodents (such as mice and rats). It will be appreciated that a particularly preferred subject according to the invention is a human subject, such as a human suffering from (or at risk of suffering from) a disorder, disease or condition, for example a human patient.

As used herein, "therapy" is synonymous with treating a disease, disorder or condition, which includes reducing symptoms of the disease, disorder or condition, inhibiting progression of the disease, disorder or condition, causing regression of the disease, disorder or condition and/or curing the disease, disorder or condition.

In preferred embodiments, a "treatment" in the present invention, and in particular, is also meant to include therapy, e.g. therapeutic treatment, as well as prophylactic or suppressive measures for a disease (or disorder or condition). Thus, for example, successful administration of a compound (or pharmaceutical composition) of the invention prior to onset of the disease results in treatment of the disease. "Treatment" also encompasses administration of a compound (or pharmaceutical composition) of the invention after the appearance of the disease in order to ameliorate or eradicate the disease (or symptoms thereof). Administration of a compound (or pharmaceutical composition) of the invention after onset and after clinical symptoms, with possible abatement of clinical symptoms and perhaps amelioration of the disease, also comprises treatment of the disease. Those "in need of treatment" include subjects (such as a human subject) already having the disease, disorder or condition, as well as those prone to or suspected of having the disease, disorder or condition, including those in which the disease, disorder or condition is to be prevented.

The cell that is sensitised to the cell-mediated immune response is involving TRAIL, suitably, one involved with the proliferative disorder (eg, a cell associated with the proliferative disorder) (in the subject), which in certain embodiments such cell is one involved in the proliferative disorder (eg, a cell that is abnormally proliferating, such as one that is over-proliferating). For example, such cell may be a cell characterised by loss of normal controls that affect its growth and cell division, such as a cell of a neoplasm or tumour. In particular embodiments, such cell may be a cancerous cell or one that is derived form or is a cell of a cancer or tumour. In other embodiments, such cell may be skin cell, such as one showing hyperproliferation such as one involved in psoriasis, Reiter's syndrome, pityriasis rubra pilaris or scleroderma.

A cell may be "involved with a proliferative disorder" if, for example, it is associated therewith, such as it being a causative factor in such proliferative disorder or if it is affected by such proliferative disorder. In particular a cell is "involved with a proliferative disorder" if the cell is characterised by an abnormal proliferation such as abnormal cell growth or cell division, and if the abnormal cell growth or cell division is part of the pathology of, or causative for, the proliferative disease. A cell "involved with a proliferative disorder", in those embodiments wherein the proliferative disorder is a tumour or cancer, can as a non-limiting example, be a tumour (or cancer) cell, or a cell of derived from (tissue) of such tumour or cancer; in particular of a solid tumour.

In certain embodiments, a compound of the invention may inhibit SIK3 in the cell involved with the proliferative disorder (eg the tumour cell). In particular of such embodiments, the compound may inhibit SIK3 in such cell preferentially to inhibiting SIK1 and/or SIK2 in such cell; and/or may inhibit SIK3 in such cell preferentially to inhibiting SIK1 and/or SIK2 and/or SIK3 in one or more types of immune cells. For example, a compound of the invention may inhibit SIK3 in the cell involved with the proliferative disorder (eg the tumour cell) preferentially to inhibiting SIK1 and/or SIK2 and/or SIK3 in macrophages and/or dendritic cells (in particular, those capable of or producing IL-10).

A compound (or pharmaceutical composition) of the invention (or a compound used in the invention) may be administered to the subject, in particular in an amount (such as a dose) that is effective to, inhibit SIK3 and/or that is effective to sensitise the cells involved with the proliferative disorder to TRAIL induced apoptosis and/or the cell-mediated immune response involving TRAIL. Suitable amounts, formulations and means for such administration are described elsewhere herein.

In particular embodiments, a compound (or pharmaceutical composition) of the invention (or a compound used in the invention) is administered in an amount (such as a therapeutically effective amount) that is effective to reduce activity of SIK3, preferably of SIK3 in (of) the cells involved with the proliferative disorder. In such embodiments, a "therapeutically effective amount" of the compound (or pharmaceutical composition) can be an amount that is capable to reduce the activity of the SIK3 to an applicable level, but that does not lead to significant (eg intolerable) side effects or over-dosage in respect of other activities of the compound (or pharmaceutical composition).

Preferably, the activity of SIK3 is effectively inhibited (reduced), preferably referring to the SIK3 kinase in (of) the cells involved with a proliferative disorder. For example, an "effective" inhibition (or reduction) may include one where the activity is lowered by a degree (or to a level) that has a physiological effect (eg to a therapeutically effective level), such as a reduction by about 10%, 20%, 50%, or more than 50% such as 70% or 90% of activity of the respective kinase. In respect of SIK3, one of such reductions may be desirable to elicit a therapeutic response.

The term "immune cell" is art recognised to describe any cell of an organism involved in the immune system of such organism, in particular of a mammal such as a human. Leukocytes (white blood cells) are immune cells that are involved in the innate immune system, and the cells of the adaptive immune system are special types of leukocytes, known as lymphocytes. B cells and T cells are the major types of lymphocytes and are derived from hematopoietic stem cells in the bone marrow. B cells are involved in the humoral immune response, whereas T cells are involved in cell-mediated immune response. In preferred embodiments of the invention, the immune cell can be a myeloid cell eg a T cell, and in particular (such as when an increase in cell-mediated immune response is required, such as to treat a cancer) the T cell can be a cytotoxic T cell (also known as TC, cytotoxic T lymphocyte, CTL, T-killer cell, cytolytic T cell, CD8+ T-cell or killer T cell). A CTL is a T-cell that is involved in the killing of cancer cells, cells that are infected (particularly with viruses), or cells that are damaged in other ways. Other preferred immune cells for such embodiments can include Tumour-Infiltrating Lymphocytes (TILs). TILs are white blood cells that have left the bloodstream and migrated into a tumour. Typically, TILs are a mix of different types of cells (eg, T cells, B cells, NK cells, macrophages) in variable proportions, T cells being the most abundant cells. TILs can often be found in the stroma and within the tumour itself, and are implicated in killing tumour cells. The presence of lymphocytes in tumours is often associated with better clinical outcomes.

The term "cell-mediated immune response", as used herein, may include, but is not limited to, a response in a host organism involving, utilising, and/or promoting any one or combinations of T cell maturation, proliferation, activation, migration, infiltration and/or differentiation, and/or the activation/modulation/migration/infiltration of a macrophage, a natural killer cell, a T lymphocyte (or T cell), a helper T lymphocyte, a memory T lymphocyte, a suppressor T lymphocyte, a regulator T lymphocyte, and/or a cytotoxic T lymphocyte (CTL), and/or the production, release, and/or effect of one or more cell-secretable or cell-secreted factor such as a cytokine or autocoid (in particular a pro-inflammatory cytokine such as TNF), and/or one or more components of any of such processes (such as a cytokine or autocoid, particular a pro-inflammatory cytokine such as TNF). The term "cell-mediated immune response," as used herein, may include a cellular response involving a genetically engineered, *in-vitro* cultured, autologous, heterologous, modified, and/or transferred T lymphocyte, or it may include a cell-secretable or cell-secreted factor (such as a cytokine or autocoid, in particular a pro-inflammatory cytokine such as TRAIL) produced by genetic engineering. A cell-mediated immune response is preferably not a humoral immune response, such as an immune response involving the release of antibodies. In certain embodiments, in particular when the proliferative disorder is a cancer or tumour, the cell-mediated immune response is an anti-tumour cell-mediated immune response. For example, one that leads to a reduction in tumour (cell) growth, such as a cytotoxic cell-mediated immune response (such as a cytotoxic T cell and/or TNF exposure) that kills cells of the cancer or tumour.

In certain embodiments, the cell-mediated immune response may be mediated by a cell, such as an immune cell, capable of secreting (eg secreting) pro-inflammatory cytokine TRAIL, and probably, a proinflammatory cytokine as one selected from the group consisting of: interleukin-1 (IL-1), IL-8 and IL-12, tumour necrosis factor (TNF), interferon gamma (IFN-gamma), and granulocyte-macrophage colony stimulating factor. In particular of such embodiments, the pro-inflammatory cytokine is tumour necrosis factor (TNF) [alpha].

In certain embodiments, the cell mediated immune response shall not involve TNF.

In all aspects of the invention, the cell-mediated immune response may be a cell-secretable or cell-secreted factor (such as a cytokine or autocoid), in particular one secretable or secreted by an immune cell. In particular of such embodiments, the cell-mediated immune response is a pro-inflammatory cytokine, in particular TRAIL.

The terms "sensitising", "sensitisation" and "to sensitise" (and the like), as used herein in the context of cell(s) being sensitised to TRAIL mediated apoptosis and/or to a cell-mediated immune response involving TRAIL, will be understood by the person of ordinary skill, and include the meaning that such cells can exhibit an increased susceptibility to one or more TRAIL involving effect (eg a treatment effect such as induction of apoptosis by TRAIl in a cell involved with the proliferative disorder) that the cell-mediated immune response involving TRAIL may have on such cells. In particular, cells that are so sensitised may, when in the presence of (eg exposed to) a cell-mediated immune response involving TRAIL, be killed more easily (such as more rapidly, a greater proportion of cells dying or being killed and/or upon a lower amount or exposure of the cell-mediated immune response) than analogous cells that have not been so "sensitised" to preferably TRAIL induced apoptosis. For example, cell(s) so sensitised may be induced into cell-death (eg apoptosis) upon exposure to a lower number of T cells or to a lower concentration of TRAIL (such as about 10%, 20%, 30% 40%, 50% or more than 50% fewer T cells or lower concentration of TRAIL). Methods to determine whether such cells have been sensitised (and by which degree) to cell-mediated immune responses are described herein, such as in the examples. Accordingly, in certain embodiments of the present invention, cells involved with the proliferative disorder may be sensitised to cell-death/killing (eg by entry into apoptosis) by a cell-mediated immune response (such as CTL or a proinflammatory cytokine eg TRAIL).

As used herein, the term "TRAIL" refers to the tumor necrosis factor (TNF)-related apoptosis-inducing ligand, also known as Apo-2 ligand (Apo2L), which is a member of the cytokine superfamily. By cross-linking TRAIL-Receptor 1 (TRAIL-R1) or TRAIL-Receptor 2 (TRAIL-R2), also known as death receptors 4 and 5 (DR4 and DR5, respectively), TRAIL has the capability to induce apoptosis in a wide variety of tumor cells while sparing vital normal cells. The discovery of this unique property led to the development of TRAIL-based anticancer agents.

The terms "TRAIL-R1", "DR4" and "DR4 receptor" are used herein interchangeably. They refer to the full length TRAIL receptor sequence and soluble, extracellular domain forms of the receptor described in Pan et al., Science, 1997, 276: 111-113; WO98/32856; U.S. Pat. No. 6,342,363; and WO99/37684.

The terms "TRAIL-R2", "DR5" and "DR5 receptor" are used herein interchangeably. They refer to the full length TRAIL receptor sequence and soluble, extracellular domain forms of the receptor described in Sheridan et al., Science, 1997, 277: 818-821; Pan et al., Science, 1997, 277: 815-818; U.S. Pat. Nos. 6,072,047; 6,342,369; WO98/51793; WO98/41629; Screaton et al., Curr. Biol., 1997, 7: 693-696; Walczak et al., EMBO J., 1997, 16: 5386-5387; Wu et al., Nature Genetics, 1997, 17: 141-143; WO98/35986; WO98/46643; WO99/02653; WO99/09165; and WO99/11791.

The term "TRAIL-based anticancer agents", as used herein, refers to any molecule or compound that interacts or interferes with TRAIL normal activity to produce a therapeutic effect. The therapeutic effect may result from any of a large variety of action mechanisms. Examples of TRAIL-based anticancer agents include, in particular, TRAIL receptor ligands and TRAIL receptor agonists.

The term "TRAIL receptor ligand", as used herein, refers to TRAIL, recombinant TRAIL, TRAIL fragments and TRAIL analogues that act as ligands to one of the TRAIL receptors (i.e., that selectively bind to one of the TRAIL receptors to form a complex), in particular to TRAIL-R1 (DR4) and/or TRAIL-R2 (DR5).

The term "TRAIL receptor agonist" refers to any molecule or compound that partially or fully enhances, stimulates or activates one or more biological activities of TRAIL-R1 or TRAIL-R2, and biologically active variants thereof, whether in vitro, in situ, in vivo or ex vivo as a result of its direct binding to one or both of these receptors, which causes receptor activation or signal transduction. In the practice of the present invention, the biological activity of TRAIL-R1 or TRAIL-R2 that is partially or fully enhanced, stimulated or activated is apoptosis.

The ligands and agonists of the TRAIL receptors that are useful in the practice of the methods of treatment of the present invention are typically TRAIL peptides or mimics, such as TRAIL fragments, variants or fusions thereof, which may optionally be linked to a conjugate molecule that extends the in vivo half-life of the TRAIL conjugate compared to the TRAIL fragments, variants or fusions in the absence of the conjugate molecule. Nucleic acid and amino acid sequences for human TRAIL are known in the art. Preferably, the TRAIL fragment is a soluble TRAIL. Endogenous, full-length TRAIL includes a cytoplasmic domain, a transmembrane domain, and an extracellular domain. Typically, soluble TRAIL is the extracellular domain of TRAIL or a functional fragment thereof or variant thereof that can agonize signalling through TRAIL-R1 or TRAIL-R2. Dulanermin (Apo1L/TRAIL), for example, is a recombinant human TRAIL which targets both TRAIL-R1 and TRAIL-R2 (see WO 2009/140469). Examples of TRAIL conjugates are known in the art (Kim et al., Bioconjugate Chem., 2011, 22: 1631-1637; Chae et al., Molecular Cancer Therapeutics, 2010, 9: 1719-1729). Examples of TRAIL analogues are known in the art, such as for example DR4-selective mutants of wild-type TRAIL (Tur et al., J. Biol. Chem., 2008, 283: 20560-20568), DR5-selective mutants of wild-type TRAIL (van der Sloot et al., PNAS USA, 2006, 103: 8634-8639) and monovalent, divalent and trivalent TRAIL-mimicking peptides (Pavet et al., Cancer Research, 2010, 70: 1101-1110). Examples of TRAIL fusion proteins are known in the art (Gieffers et al., Molecular Cancer Therapeutics, 2013, 12: 27357; Wahl et al., Hepatology, 2013, 57: 625-636). A TRAIL agonist may be an agonistic antibody that binds to the TRAIL-R1 (DR4) receptor or to the TRAIL-R2 (DR5) receptor. Agonistic antibodies, that bind to and activate the receptor, mimicking the natural ligand, TRAIL, are also called "anti-TRAIL receptor antibodies". Agonistic antibodies that bind to TRAIL-R1 (DR4) include mapatumumab (HGS-ETR1), a fully humanized monoclonal antibody. Agonistic antibodies that bind to TRAIL-R2 (DR5) include lexatumumab (HGS-RTR2), drozitumab (Apomab/PRO95780), conatumumab (AMG 655), tigatuzumab (CS-1008/TRA-8), HGSTR2J/KMTRS and LBY-135. Lexatumumab, drozitumab, and conatumumab are fully human IgG1 antibodies, while tigatuzumab is a humanized IgG1 antibody and LBY135 is a chimeric mouse/human antibody. TRAIL receptors and antibodies that bind to TRAIL receptors are described in U.S. Pat. Nos. 6,455,040 (DR5); 6,743,625 (DR5); 6,433,147 (DR4), and 6,902,910 (DR4). Other examples include anti-TRAIL-R receptor antibodies (U.S. Pat. No. 7,115,717); anti-TRAIL-R4 (U.S. Pat. Publ. No. 2002097033); anti-TRAIL-R antibodies (U.S. Pat. Publ. No. 20060062786); anti-DR5 mapatumumab and drozitumab (U.S. Pat. Publ. No. 2014161845); anti-DR5 lexatumumab and tigatuzumab (U.S. Pat. Publ. No. 2014159562), and LBY 135 conatumumab U.S. Pat. Publ. No. 2013148877).

Thus, the present invention provides for the use of at least one SIK3 inhibitor described herein to improve the sensitivity of a cancer patient to TRAIL receptor agonist therapy or a tumor which is resistant to endogenous TRAIL induced apoptosis. As used herein, the term "TRAIL receptor agonist therapy" refers to a therapy (or method of treatment) wherein a TRAIL-based anticancer agent (or TRAIL receptor ligand or agonist) is administered to the subject treated. The present invention also provides a method of improving the sensitivity of a cancer patient to TRAIL receptor agonist therapy, said method comprising steps of: (1) identifying a cancer patient as a candidate for TRAIL receptor agonist therapy or a cancer patient treated using a receptor agonist therapy, and (2) administering to the patient an effective amount of at least one of the SIK3 inhibitors of the invention and an effective amount of at least one TRAIL receptor ligand or agonist, wherein the at least one of the SIK3 inhibitors is administered before, during or after administration of the at least one TRAIL receptor ligand or agonist.

In certain embodiments, the cell-mediated immune response can be mediated by a pro-inflammatory cytokine-secreting cell, such as a lymphocyte (eg a T cell), in particular a cytotoxic T lymphocyte (CTL).

In particular embodiments, the cell-mediated immune response may induce killing (eg cell-death, such via apoptosis) of cells involved with the proliferative disorder. For example, the treatment (method) may comprise (eg may involve) that (or be mediated by) the cell-mediated immune response induces such killing of cells involved with the proliferative disorder.

The cells involved with the proliferative disorder may be killed (eg induced into cell death) by one or more cytotoxic processes, in particular those that are endogenous to such cell such as programmed cell death (PCD). Cell death processes may include, but are not limited to, necrosis (in particular necroptosis), apoptosis, anoikis, autophagy, ferroptosis, mitotic catastrophe and activation-induced cell death. In certain preferred embodiments, the cells involved with the proliferative disorder (eg the tumour cells) are induced into apoptosis by the cell-mediated immune response (eg by TRAIL as described above). In a further embodiment, a compound (or pharmaceutical composition) of the invention is administered to not kill such cells in the absence of the cell-mediated immune response (eg in the absence of TRAIL). In particular of such further embodiments, the compound (or pharmaceutical composition) may be administered in an amount (eg in a dose) that is not effective to kill such cells in the absence of the cell-mediated and TRAIL dependent immune response. The examples herein, describe various assays by which an amount of a compound (or pharmaceutical composition) of the invention may be determined that is effective to kill such cells only, or preferentially, in the presence of the cell-mediated immune response.

In other particular embodiments, the cell-mediated immune response may involve at least one immune cell effector molecule, in particular an effector molecule that is secretable or secreted by an immune cell. In particular of such embodiments, the effector molecule is preferably TRAIL, or a TRAIL agonist.

In certain embodiments, the effector molecule is not a cell effector molecule selected from Fas ligand (FasL or CD95L) and TNF).

In particular embodiments of the invention, a compound (or pharmaceutical composition) of the invention may be administered to the subject (eg in an amount or dose effective) with the intent to (or so as to) (effectively) sensitise cells involved with the proliferative disorder to killing induced by TRAIL. For example, the compound (or pharmaceutical composition) may be administered in a therapeutically effective amount, such as an amount effective to sensitise the cells involved with the proliferative disorder to killing (cell-death) induced by TRAIL.

For example, a compound (or pharmaceutical composition) of the invention may be administered to the subject (for example, in an amount or dose effective) to induce apoptosis of such cells mediated by TRAIL, such as when such cells are in the presence of or contacted with TRAIL. In further embodiments, the compound (or pharmaceutical composition) of the invention may be administered to the subject (eg in an amount or dose effective) to induce a reduced amount of cytotoxicity (eg apoptosis) - such as to not induce killing (eg apoptosis) of such cells - in the absence of TRAIL; for example the compound (or pharmaceutical composition) may be administered in an amount or dose that is - not as effective in cytotoxicity (eg apoptosis) - such as being not effective to induce such killing - in the absence of TRAIL.

As described in PCT/EP2018/060172 (WO 2018/193084), the inhibition of SIK3 is associated with a number of key biological processes or phenotypes, including those surprisingly involved in the control and/or triggering of cytotoxic process innate to cells, such as apoptosis. For example, tumour cells can be sensitised to the apoptotic/cytotoxic effects of TNF by the inhibition of SIK3, acting through pathways and components thereof including liver kinase B1 (LKB1, STK11 or NY-REN-19), histone deacetylase 4 (HDAC4), nuclear factor kappa-light-chain-enhancer of activated B cells (NF-kappaB), and pro-apoptotic genes regulated by NF-kappaB such as Caspase 8 and Caspase 9. Also c-Jun N-terminal kinase (JNK) is a signalling component associated with sensitisation to the apoptotic/cytotoxic effects of TNF by the inhibition of SIK3. While it was generally thought that the effect is specific for TNF, the present invention surprisingly shows that cells involved with a proliferative disorder may be sensitized to TRAIL induced apoptosis or to a cell mediated immune response involving TRAIL, by inhibiting SIK3 in such cell involved with the proliferative disorder.

The term "associated with", in the context of this embodiment (and other embodiments, where applicable) can mean that two components, variables, effects or phenotypes are interrelated with each other, and/or that they are related to (eg correlated to) each other, and/or that there is a causative link between a first and a second component, variable, effect or phenotype (such as the second is in response to the first, the second is a consequence of the first, or the second is caused by the first).

Accordingly, in one such embodiment, administration of a compound (or pharmaceutical composition) of the invention can associate with impairment of NF-kappaB activity (eg, by an enhancement or increase in translocation of NF-kappaB out of the nucleus) in cells involved with the proliferative disorder.

In particular of such embodiments, such impairment of NF-kappaB activity (eg, by an enhancement or translocation of NF-kappaB out of the nucleus) may be associated with (activated) TRAIL- and/or a TRAIl receptor-mediated signalling in such cells.

In certain embodiments, a compound (or pharmaceutical composition) of the invention may be administered to the subject (eg in an amount or dose effective) to impair or inhibit NF-kappaB activity in the cells involved with the proliferative disorder, for example to enhance or increase translocation of NF-kappaB out of the nucleus of such cells. For example, the compound (or pharmaceutical composition) may be administered to the subject in a (eg, therapeutically effective) amount being effective to (effectively) impair NF-kappaB activity in cells involved with the proliferative disorder, in particular in an amount effective to (effectively) enhance or increase translocation of NF-kappaB out of the nucleus of the cells involved with the proliferative disorder.

In alternative or further embodiments, administration of a compound (or pharmaceutical composition) of the invention may be associated with an increase in (eg, the compound (or pharmaceutical composition) is administered, such as in an amount or dose effective, to increase) activity of class II (eg IIa) HDACs, eg HDAC4, in the cells involved with the proliferative disorder, for example its translocation or localisation to or its activity in the nucleus of such cells; and in particular upon TRAIL- and/or a TRAIL receptor-mediated signalling in such cells.

In other alternative or further embodiments, administration of a compound (or pharmaceutical composition) of the invention may be associated with de-acylation of nuclear NF-kappaB (eg de-acylation at its p65 subunit) and/or decreased transactivation of one or more anti-apoptotic factors, in particular upon TRAIL- and/or TRAIL receptor-mediated signalling in the cells involved with the proliferative disorder. For example, the compound (or pharmaceutical composition) may be administered (such as in an amount or dose effective) to cause de-acylation of nuclear NF-kappaB (eg at its p65 subunit) and/or decreased transactivation of one or more anti-apoptotic factors.

In another alternative or further embodiment, administration of a compound (or pharmaceutical composition) of the invention may be associated with an increase in (eg the compound (or pharmaceutical composition) is administered, such as in an amount or dose effective, to increase) cleavage of Caspase 8 and/or Caspase 9 in the cells involved with the proliferative disorder, in particular upon TRAIL- and/or TRAIL receptor-mediated signalling in such cells.

In yet other alternative or further embodiments, administration of a compound (or pharmaceutical composition) of the invention may be associated with a reduction in the transcription of one or more anti-apoptotic factors, in particular upon TRAIL- and/or TRAIL receptor-mediated signalling in the cells involved with the proliferative disorder, for example the reduction of the transcription of one or more NF-kappaB target genes in such cells. In particular, the compound (or pharmaceutical composition) may be administered (eg in an amount dose effective) to reduce the transcription of one or more such anti-apoptotic factors, in particular upon TRAIL- and/or TRAIL receptor-mediated signalling in the cells involved with the proliferative disorder.

In **a further aspect,** and as may be further described, defined, claimed or otherwise disclosed herein, the invention relates to **a method for the sensitisation of cells** involved with a proliferative disorder to TRAIL mediated apoptosis and/or to cell-mediated immune response involving TRAIL, the method comprising exposing (eg contacting) the cells involved with a proliferative disorder to a compound (or pharmaceutical composition) of the invention. Such a method may, typically, be practiced as an in-vitro and/or ex-vivo method.

In a particular embodiment, the cell-mediated immune response comprises killing the cells involved with a proliferative disorder, such as where said killing involves (eg, is mediated, is or supported by) TRAIL or TRAIL receptor (such as DR4 or DR5) therefore TRAIL or TRAIL receptor-mediated signalling. For example, the killing of such cells may involve apoptosis of such cells induced by TRAIL, DR4- and/or DR5-mediated signalling. Within this and the other applicable embodiments of the various aspects of the invention, DR4- and/or DR5-mediated signalling may be triggered (eg activated) by any appropriate triggering molecule, such as TRAIL, a variant of TRAIL and or a DR4 or DR5 agonist; in particular by exposing (eg by contacting) the cells associated with the proliferative disorder to the triggering molecule (eg TRAIL, TRAIL variant or TRAIL receptor (DR4 or DR5) agonist). Such exposure can lead to the triggering molecule (eg TRAIL, TRAIL variant or TRAIL receptor (DR4 or DR5) agonist) binding to DR4 and/or DR5 and, in particular the triggering (eg activation) of TRAIL signalling.

In **a yet further aspect,** and as may be further described, defined, claimed or otherwise disclosed herein, the invention relates to **a method for the killing of cells** involved with a proliferative disorder, the method comprising exposing (eg contacting) the cell involved with the proliferative disorder to: (i) TRAIL, TRAIL variant or TRAIL receptor (DR4 or DR5 ) agonist; and exposing (eg contacting) the cells involved with the proliferative disorder to (ii) a compound (or pharmaceutical composition) of the invention. As will be appreciated by the person or ordinary skill, such a method may, typically, be practiced as an in-vitro and/or ex-vivo method.

In **a related aspect,** the invention relates to **a compound (or pharmaceutical composition)** of the invention **for use in the treatment of a proliferative disease** involving the killing of a cell involved with the proliferative disorder, the treatment comprising exposing such cell to: (i) TRAIL, TRAIL variant or TRAIL receptor (DR4 or DR5 ) agonist; and (ii) a compound (or pharmaceutical composition) of the invention.

In particular embodiments of such aspects, the killing of the cell involved with the proliferative disorder is mediated by sensitising such cell to a cell-mediated immune response, in particular by inducing sensitivity to apoptosis of such cell that involves (eg, is mediated, is or supported by) TNF, TNFR2 and/or TNFR1-mediated signalling.

The cell(s) involved with the proliferative disorder may be exposed to the TRAIL, TRAIL variant or TRAIL receptor (DR4 or DR5 ) agonist by contacting the cell to such triggering molecule; and/or such cell(s) may be exposed to a compound (or pharmaceutical composition) of the invention by contacting (or introducing into) such cell(s) with a compound (or pharmaceutical composition) of the invention. The amounts (or dose) of (i) TRAIL, TRAIL variant or TRAIL receptor (DR4 or DR5 ) agonist; and/or (ii) a compound (or pharmaceutical composition) of the invention are, typically, effective amounts; that is amounts (or doses) that are effective in, for example, sensitising the cell(s) to (such as killing such cell(s) by) apoptosis induced by TRAIL, TRAIL variant or TRAIL receptor (DR4 or DR5 ) signalling. Elsewhere are disclosed suitable amounts of these active agents (or ways to determine them) that may be incorporated in these aspects of the invention; as are further particular characteristics of the compound (or pharmaceutical composition) of the invention. Accordingly, in certain embodiments: (i) TRAIL, TRAIL variant or TRAIL receptor (DR4 or DR5) agonist; and (ii) a compound (or pharmaceutical composition) of the invention, can be administered to a subject suffering from the proliferative disorder (eg, the treatment can comprise the administration of: (i) TRAIL, TRAIL variant or TRAIL receptor (DR4 or DR5) agonist; and (ii) a compound (or pharmaceutical composition) of the invention, can be administered to the subject).

The cell(s) involved with the proliferative disorder may be one as described elsewhere herein, and in particular such cell(s) may be cancerous or tumour cell. For example, such cell(s) may be one that is of, or derived from, a solid tumour.

In certain embodiments of these aspects, the method is an in vitro (and/or ex-vivo) method. In alternative embodiments of such methods, the cell(s) involved with the proliferative disorder (such as tumour cells) is present in such subject, in particular in a subject in need of treatment thereof.

In further embodiments of the methods of these aspects, the (treatment) effect of such method (eg, on the cell(s) involved with the proliferative disorder) can be mediated by (eg, the treatment may comprise, involve or be mediated by) inhibiting SIK3; in particular, by inhibiting the function and/or activity of SIK3 protein (eg, of phosphorylated SIK3 protein, and/or as described elsewhere herein). In particular, in such embodiments, the SIK3 activity is (eg, effectively) reduced, such as reduced to a therapeutically effective level.

In certain embodiments of such methods, in the absence of (eg such effective amount or dose of) a compound (or pharmaceutical composition) of the invention, the cell(s) involved with the proliferative disorder (such as the tumour cell(s)) are not killed or induced to enter apoptosis (for example, they proliferate) upon TRAIL, TRAIL variant or TRAIL receptor (DR4 or DR5) signalling and/or exposure to a (eg, the effective amount or dose of) TRAIL, TRAIL variant or TRAIL receptor (DR4 or DR5) agonist.

As described above, in certain embodiments of these methods, a compound (or pharmaceutical composition) of the invention may inhibit SIK3 in (of) the cell(s) involved with the proliferative disorder (eg tumour cells). In particular of such embodiments, the compound (or pharmaceutical composition) may inhibit SIK3 in (of) such cell(s) preferentially to inhibiting SIK1 and/or SIK2 in (of) such cell; and/or may inhibit SIK3 in such cell preferentially to inhibiting SIK1 and/or SIK2 and/or SIK3 in (of) one or more types of immune cells. For example, a compound (or pharmaceutical composition) of the invention may inhibit SIK3 in (of) the cell(s) involved with the proliferative disorder (eg tumour cells) preferentially to inhibiting SIK1 and/or SIK2 and/or SIK3 in (of) macrophages and/or dendritic cells (in particular, those capable of or producing IL-10). In particular embodiments, the (treatment) effect is mediated by (eg, the treatment comprises, involves, is by or is mediated by) inhibition of SIK3 in (of) the cell(s) involved with the proliferative disorder (eg a tumour cell); and in further of such embodiments, the (treatment) effect is not mediated by (or the effect is mediated by not) (eg, the treatment does not comprise, involve or is not mediated by) inhibiting SIK2, in particular SIK2 in/of other cells (such as those involved with the proliferative disorder or immune cells), and/or the (treatment) effect is not mediated by (or the effect is mediated by not) inhibiting SIK1 (eg, the treatment does not comprise, involve or is not mediated by inhibiting SIK1), in particular SIK1 in/of other cells (such as those involved with the proliferative disorder or immune cells).

Accordingly, in one embodiment, the SIK3 of (eg, in) the cell(s) involved with the proliferative disorder is inhibited (eg, by a compound or pharmaceutical composition of the invention). In another (or further) embodiment, another kinase (eg SIK2, in particular SIK2) of (eg in) immune cells - such as CTLs - is inhibited to a lesser extent than SIK3 (eg in the cell(s) involved with the proliferative disorder). In yet another (or further) embodiment SIK1, in particular SIK1 of (eg in) immune cells - such as CTLs - is inhibited to a lesser extent than such SIK3.

In certain of such embodiments, one or more of the kinases selected from the list consisting of: SIK3, SIK1, SIK2, JAK1, RET, ERBB4 PDGFR-alpha, and EPHB2, is inhibited (eg, by a compound or pharmaceutical composition of the invention) to a lesser extent than one or more of the kinases selected from the list consisting of: ABL1, SRC, BCR-ABL, LCK, LYN, YES, FYN, KIT and FLT3.

In certain of such embodiments, one or more of the kinases selected from the list consisting of: PDGFR-alpha, TGFB-R1, B-RA, p38-beta, ACV-R1, BMPR1A and RET, is inhibited (eg, by a compound or pharmaceutical composition of the invention) to a lesser extent than one or more of the kinases selected from the list consisting of: EPHA2, EPHA4, CSF1R, HCK and ACK1.

In certain of such embodiments, one or more of the kinases selected from the list consisting of: NEK11, WEE1, WNK2, Aurora-A, Aurora-B and TBK1, is inhibited (eg, by a compound or pharmaceutical composition of the invention) to a lesser extent than one or more of the kinases selected from the list consisting of: ABL1, SRC, BCR-ABL, LCK, LYN, YES, FYN and KIT.

A given kinase (such as SIK1 or SIK2) may be inhibited to a "lesser extent" than another kinase (such as SIK3) if, for example, the other kinase (such as SIK3) is inhibited by an amount greater than about 2 fold more than the given kinase, such as by an amount greater than about 5, 10, 20, 50, 75 or 100-fold more than the given kinase. In particular, the other kinase (such as SIK3) may be inhibited by an amount between about 5 and 20 fold, 20 and 50 or 50 and 100 fold more than the given kinase. For example, the SIK3 (ie, the other kinase) may be inhibited between about 20 and 50 fold more than SIK1 and/or SIK2 (ie, a given kinase). By way of example, a compound (or pharmaceutical composition) of the invention may inhibit the other kinase (eg SIK3) by 80% (ie, to have only 20% of its uninhibited activity) but inhibit the given kinase (eg SIK1) by only 4% and SIK2 by only 8%. Accordingly, the other kinase (eg SIK3) is inhibited about 20-fold more than the given kinase (eg SIK1) and 10-fold more than another given kinase (eg SIK2). In particular embodiments, the other kinase (eg SIK3) may be inhibited to about the same extent as eg SIK1 (eg between about 2 to 53 fold of each other), and eg SIK2 is inhibited to a lesser extent that either (or both) of eg SIK3 and SIK1: For example, in such embodiments, eg SIK3 and SIK1 are inhibited by between about a 20 and 50 fold more than eg SIK2 (eg in immune cells) is inhibited.

Certain cells involved with the proliferative disorder (eg tumour cells) may, in certain embodiments, be expected to be more susceptible to the sensitising effects of a compound (or pharmaceutical composition) of the invention in the various aspects of the invention. For example, such cells may be those that exhibit (eg are subject to) activation of DR4 and/or DR5 signalling. In certain embodiments, such cells are those that express DR4 and/or DR5,. Accordingly, in certain embodiments, such cells are distinguished or characterised by activated DR4- and/or DR5-signalling (or the subject is distinguished or characterised by having cells involved with the proliferative disorder - eg tumour cells - that are so distinguished or characterised). The person of ordinary skill will know techniques for determining the status of DR4- and/or DR5-activation in such cells (such as of the subject). For example, by detecting or monitoring one or more down-stream protein in the DR4- and/or DR5-signalling pathways. Such proteins are described elsewhere herein, and include certain caspases, NF-kappaB and/or HDAC4.

In **one related aspect,** the invention relates to **a method for the treatment of a proliferative disorder** (such as a tumour) in a subject, the (treatment) method comprising administering a compound (or pharmaceutical composition) of the invention to the subject, by inhibiting a kinase/key-kinase (eg SIK3), wherein cells involved with the proliferative disorder are characterised by (eg exhibit or are subject to) activated DR4 and/or DR5 signalling. In another related aspect, the invention relates to a compound (or pharmaceutical composition) of the invention for use in the treatment of a proliferative disorder, wherein cells involved with the proliferative disorder are distinguished or characterised by (eg exhibit or are subject to) activated DR4 and/or DR5 signalling.

In certain embodiments of the various aspects of the invention, cells involved with the proliferative disorder are those exposed to an appropriate triggering or activating molecule, such as TRAIL, a variant of TRAIL and or an agonist of DR4- or DR5-signalling, in particular are exposed to an effective amount of such triggering or activating molecule.

In particular embodiments, when the triggering or activating molecule is TRAIL, it is human TRAIL. In certain of such embodiments, the TRAIL is recombinant human TRAIL (rHuTRAIL). However, in other embodiments the TRAIL is endogenous TRAIL, such as that is produced by or otherwise present in the subject (eg the human patient).

Accordingly, **in a related aspect,** the invention can relate to **a method for the treatment of a proliferative disorder** (or a compound (or pharmaceutical composition) of the invention for use in such a treatment) in a subject distinguished by having: (i) a plasma concentration of TRAIL that is sufficient to induce apoptosis in cells involved with the proliferative disorder; and/or (ii) an intratumoural concentration of TRAIL sufficient to induce apoptosis in the tumour, the treatment method comprising administering a compound (or pharmaceutical composition) of the invention to the subject, wherein the compound (or pharmaceutical composition): (a) inhibits a kinase/key-kinase (eg SIK3) in cells involved with the proliferative disorder; and/or (b) sensitises cells in the subject involved with the proliferative disorder to a cell-mediated immune response.

In particular of such embodiments, the amount (or dose) of a compound (or pharmaceutical composition) of the invention that is exposed to cells involved with the proliferative disorder, or that is administered to the subject, is related to (eg correlated to) the plasma or intratumoural concentration of TRAIL, wherein a greater amount (or dose) of the compound (or pharmaceutical composition) is exposed to such cells (or administered to such subject) in those cases of a greater plasma or intratumoural concentration of TRAIL.

In other or further embodiments, the tumour may be present in a subject having tumour-reactive T-cells in peripheral blood or bone marrow, for example as may be determined by IFN-gamma ELISPOT. In yet other or further embodiments, the tumour shows infiltration by Tregs, CD4+ Tconv and/or CD8+ T cells.

The invention hereby provides alternative combination treatment regimens based on the surprising finding of the inventors that inhibition of one or more kinase, such a one or more key-kinases, (eg SIK3) by compounds of the invention can influence the sensitivity of a cell towards the apoptotic/cytotoxic effects of TRAIL. Accordingly, in a further aspect, and as may be further described, defined, claimed or otherwise disclosed herein, the invention relates to a method for the treatment of a proliferative disorder in a subject, the method comprising exposing (eg contacting) cells involved with the proliferative disorder in the subject to: (i) TRAIL, a TRAIL variant and/or an agonist of DR4- or DR5-signalling; and exposing (eg contacting) the cells involved with the proliferative disorder in the subject to (ii) a compound (or pharmaceutical composition) of the invention. In certain embodiments, step (i) of such method does not comprise exposing (eg contacting) cells involved with the proliferative disorder in the subject to a TRAIL variant.

In certain embodiments, the proliferative disorder and/or such cells are those of the tumour, and in other embodiments, component (i) is TRAIL, in particular human TRAIL; and/or component (i) is an agonist of DR4/DR5-signalling.

In particular embodiments, the method comprises (eg the treatment comprises, involves, is by or is mediated by) increasing the amount of TRAIL exposed to the cells involved with the proliferative disorder in the subject.

In certain embodiments of such aspects, the treatment may comprise (eg, involves, is by or is mediated by) increasing DR4- and/or DR5-signalling in (of) the cells involved with the proliferative disorder in the subject. Accordingly, in a related aspect the invention relates to a method for the treatment of a proliferative disorder in a subject, the method comprising: (i) increasing DR4- and/or DR5-signalling in (of) the cells involved with the proliferative disorder; and (ii) exposing (eg contacting) the cells involved with the proliferative disorder in the subject to a compound (or pharmaceutical composition) of the invention.

In particular the method can, for example, be effected though the consequence(s) of inhibition of a kinase (eg a key-kinase such as SIK3) (such as inhibition of the function and/or activity of phosphorylated SIK3), in particular in combination with the consequence(s) of activation of DR4- and/or DR5-signalling, such as upon binding of the TRAIL, TRAIL variant and/or agonist to DR4 or DR5.

Accordingly, the treatment effect can, in certain embodiments, involve, or be mediated (eg, caused) by, inhibiting a kinase (eg a key-kinase, such as SIK3), and/or by sensitising the cells involved with the proliferative disorder to the cytotoxic (eg apoptotic) effects of DR4- or DR5-signalling. In particular of such embodiments, the kinase/key-kinase activity may be (effectively) reduced, such as to a therapeutically effective level.

As described above, herein are also envisioned embodiments wherein a kinase, such as a key-kinase (eg SIK3) in the tumour cells is inhibited and, optionally, where one or more other kinase/key-kinase (eg SIK2 and/or SIK1) are inhibited to a lesser extent, such as such other kinase (eg SIK2 or SIK1) of immune cells.

Also as described above, herein are also envisioned embodiments wherein the treatment comprises, involves, is by or is mediated by (eg, a compound (or pharmaceutical composition) of the invention is administered in an amount, such as a therapeutically effective amount that is effective to) inhibition of a kinase/key-kinase activity such that it is (eg, effectively) reduced, such as reduced to a therapeutically effective level.

In certain embodiments of such aspect, the subject can be administered a compound (or pharmaceutical composition) of the invention and/or can be administered (the) TRAIL, an (the) TRAIL variant or an (the) agonist of DR4- or DR5-signalling.

In such embodiments, a compound (or pharmaceutical composition) of the invention and the TRAIL, TRAIL agonist or variant or DR4 or DR5 agonist can be exposed to (for example administered in) an effective amount (or dose), including in formulations or administrative routes as described elsewhere herein. In particular are envisioned embodiments where the TRAIL, TRAIL agonist or variant or DR4 or DR5 agonist is encapsulated as a liposomal or other nanoparticle formulation.

When the TRAIL, TRAIL agonist or variant or DR4 or DR5 agonist is exposed/administered and a compound (or pharmaceutical composition) of the invention is exposed/administered, then such combination treatment regimen may comprise embodiments where such exposures/administrations are concomitant. In alternative embodiments such exposures/administrations may be sequential; in particular those embodiments where a compound (or pharmaceutical composition) of the invention is exposed/administered before the TRAIL, TRAIL agonist or variant or DR4 or DR5 agonist is exposed/administered. For example a compound (or pharmaceutical composition) of the invention may be sequentially exposed/administered within about 14 days of (eg before) the other component, such as within about 10 days, 7 days, 5 days, 2 days or 1 day of (eg before) the other component; and further including where the compound (or pharmaceutical composition) may be sequentially exposed/administered within about 48 hours, 24 hours, 12 hours, 8 hours, 6 hours, 4 hours, 2 hours, 1 hours, 30 mins, 15 mins or 5 mins of (eg before) the other component.

The TRAIL, TRAIL agonist or variant or DR4 or DR5 agonist may be administered via conventional routes, such as s.c., i.v. or i.m., and on certain embodiments may be administered intratumourally or by isolated limb perfusion (ILP), such as isolated hepatic perfusion (IHP); and/or may be so administered (in particular, rHuTNF may be so administered) at a dose of between about 5 and 500 µg/m²/day. For example, TRAIL may be administered between about 25 and 250 µg/m²/day, such as between about 50 and 150 µg/m²/day or between about 75 and 100 µg/m²/day; or wherein TNF is administered up to a MTD of about 50 and 75 µg/m²/day when administered s.c. or up to a MTD of about 150 and 200 µg/m²/day when administered i.v. or i.m. Accordingly, in particular of such embodiments, TRAIL can be administered to the subject at a dose of between about 5 and 500 µg/m²/day, in particular between about 20 and 200 µg/m²/day.

In alternative embodiments, cells involved with the proliferative disorder (eg tumour cells) may be exposed to TRAIL (or increased DR4- and/or DR5-signalling) through the administration of an agent (eg to a subject harbouring such cell) that can lead to the exposure of such cells to (eg endogenous) TRAIL, or to another triggering molecule such as a variant of TRAIL or a DR4 or DR5 agonist. Such an agent may, for example, be one that is capable of inducing (eg induces) the exposure of such cells to (eg an elevated level of) TRAIL, in particular an agent that induces the exposure of such cells to TRAIL levels, such as to an effective amount of (eg endogenous) TRAIL, for example levels of plasma or intratumoural TRAIL that are greater than one or those levels described elsewhere herein.

Accordingly, the invention includes those embodiments wherein the subject is administered an agent that is capable of inducing (eg induces) the exposure of the cells involved with the proliferative disorder to (the) TRAIL, an (the) TRAIL variant or an (the) agonist of DR4- or DR5-signalling. The invention also includes those embodiments wherein the subject gets administered an agent that is capable of increasing DR4-signalling (and/or DR5-signalling) of, and/or increasing the amount of TRAIL exposed to, cells involved with the proliferative disorder in the subject.

In certain of such embodiments, the agent is a virus, in particular one that has been engineered to produce a triggering molecule being TRAIL, TRAIL agonist or variant or DR4 or DR5 agonist (especially, a virus engineered to produce human TRAIL). Further of such embodiments include those where such virus preferentially infects the cell(s) involved with the proliferative disorder (eg tumour cells) and/or preferentially produces the triggering molecule in the context of (eg when it infects) such cells. As will now be apparent, the administration of such a virus can lead to the exposure of the cell(s) involved with the proliferative disorder to such triggering molecule, and in particular to an effective amount of such a triggering molecule such as TRAIL.

When administered as an agent in such embodiments of the invention, the immune cell may be administered via adoptive cell transfer (ACT); meaning the transfer of the immune cell into the subject (eg, by infusion or other delivery techniques). Such process is, typically, conducted with the goal of improving immune functionality and characteristics in the subject, and while conventionally the transferred immune cells will have originated from the same subject, they may alternatively have been derived from another (suitable) individual.

When used in this embodiment of the invention, the immune cells may be T cells extracted from the subject, genetically modified and cultured in vitro and returned to the same subject, such as in a therapeutic method of the invention. Such genetic modification can include those that enhance the specificity or targeting of the immune cell, such as the targeting of the immune cell (eg increasing its specificity) to the cell(s) involved with the proliferative disorder (eg a tumour cell). For example, a T cell that is used in such embodiments may be modified to alter the specificity of the T cell receptor (TCR) or to introduce antibody-like recognition in chimeric antigen receptors (CARs). CAR immune cells, in particular, are envisioned for use in such embodiments. CAR immune cells are immune cells displaying engineered receptors, which graft an arbitrary specificity (eg to a tumour cell) onto an immune effector cell (eg a T cell). Typically, these receptors are used to graft the specificity of a monoclonal antibody onto a T cell; with transfer of their coding sequence facilitated by retroviral vectors. CAR T cells are a promising therapy for cancer (Song et al 2015, Oncotarget. 6:21533): using ACT, T cells are removed from an individual (typically the subject) and modified so that they express receptors specific to the patient's particular cancer. These T cells, which can then recognise the subject's cancer cells, are (re)introduced into the subject, leading to exposure of TRAIL (eg produced by the CAR T cells) to the tumour cells and hence killing of such cells, in particular such cells that are sensitised to such TRAIL-mediated cytotoxicity by exposure to (eg following administration to the subject of) a compound (or pharmaceutical composition) of the invention. Accordingly, in particular of such embodiments, the immune cells can be a CAR T cell, such as one engineered to have increased specificity to the subject's cells that are involved with the proliferative disorder (such as tumour cells).

Without being bound to theory, administration of a compound (or pharmaceutical composition) of the invention (and hence inhibition of the expression, amount, function, activity or stability of a kinase /key-kinase such as SIK3, eg in a tumour cell) prior to administration of the TRAIL, TRAIL variant or DR4 or DR5 agonist, or prior to administration of such other procedures (eg, the other agent, the cancer immunotherapy, the cancer vaccine, the antibody or the radiotherapy, is foreseen to be particularly effective in sensitising the cells involved with the proliferative disorder to the cytotoxic effects of the cell-mediated immune response.

In **another aspect,** and as may be further described, defined, claimed or otherwise disclosed herein, the invention relates to **a method for the increase of the therapeutic index of treatment with TRAIL or a TRAIL agonist** in a subject being treated therewith for a proliferative disorder (eg a cancer disease or a tumour), the method comprising administering a compound (or pharmaceutical composition) of the invention to the subject.

In **a related aspect,** the invention relates to **a method for supporting TRAIL therapy** in a subject suffering from a proliferative disorder (eg a cancer disease or a tumour), the method comprising administering a compound (or pharmaceutical composition) of the invention to the subject.

In **a further aspect,** and as may be further described, defined, claimed or otherwise disclosed herein, the invention relates to **a method for the sensitisation** of a subject suffering from a proliferative disorder (eg a cancer disease or tumour) to a therapy involving the administration of TRAIL or a TRAIL agonist to the subject, the method comprising administering a compound (or pharmaceutical composition) of the invention to the subject.

The term "sensitisation" (and the like), as used herein in the context of a subject being sensitised to a therapy (eg one involving the administration of TRAIL), will be understood by the person of ordinary skill, and includes the meaning that the subject increases susceptibility to one or more (treatment) effect - in particular an efficacy effect - that such therapy may have on the subject. In particular, a subject that is so sensitised may, when undergoing such therapy, show an increased response (such as more rapidly, a greater degree of response and/or upon a lower amount or exposure of such therapy) than an analogous subject that have not been so "sensitised".

### SIK3 Inhibitor Compounds

"Salt-inducible kinase 3" or "SIK3" (synonyms QSK and KIAA0999) is a member of a subfamily of serine/threonine protein kinases including SIK1, SIK2, and SIK3 that belong to an AMP-activated protein kinase (AMPK) family. A SIK3 protein in context of the invention is, typically, a protein kinase. Pertinent information on the human SIK3 protein is accessible on UniProt: Q9Y2K2 (Entry version 138 of 15-Mar-2017) and a SIK3 protein in context of the invention has, preferably, an amino acid sequence shown in SIK3, Entry version 138 of 15-Mar-2017 or Entry version 144 of 28-Mar-2018, which sequences are incorporated herein by reference. SIK3 is a cytoplasmatic protein with serine/threonine kinase activity which is regulated through phosphorylation of a conserved threonine residue (position 163) in the T-loop of the kinase domain by the LKB1 complex; a phosphorylation which is reported as essential for catalytic activity of SIK3 (Lizcano, J. M. et al.; EMBO J. 23, 833-843 (2004)). For the purposes of the herein disclosed invention the term "phosphorylated SIK3" shall denote a SIK3 protein that is phosphorylated substantially as SIK3 protein can be (eg is) phosphorylated by LKB1, wherein preferably such phosphorylated SIK3 comprising a phosphor-threonine at amino acid position 163. A phosphorylated SIK3 in context of the invention is an SIK3 protein that is activated in its cell-biological context. At least four protein isoforms (SIK3-001 to SIK3-004) generated by alternative splicing of the SIK3 gene product are known. The human SIK3 gene is located at chromosomal position 11q23.3 (HGNC gene Symbol Acc: HGNC:29165), and is conserved in many species such as in chimpanzee, Rhesus monkey, dog, cow, mouse, rat, chicken, zebrafish, and frog. The term SIK3 in some embodiments of the invention may also pertain to variants of the human SIK3 protein having an amino acid sequence that is substantially identical to, or of at least 80%, preferably 85%, more preferably 90, 95, 96, 97, 98, 99, or 100% sequence identity to, the amino acid sequence of SIK3 as described above, as determined using, e.g., the "Blast 2 sequences" algorithm described by Tatusova & Madden 1999 (FEMS Microbiol Lett 174: 247-250), and which (preferably) retain biological activity identical or substantially identical to the respective reference SIK3 (eg to phosphorylate one or more class II (eg IIa) HDACs, such as HDAC4). Preferred variants of SIK3 protein comprise sequence variants thereof due to sequence polymorphism between and within populations of the respective species, as well as mutations compared to the wild-type sequence of SIK3 which are located in or in close proximity to the activity loop or activation loop (T-loop) of SIK3. A preferred variant of SIK3 protein is a SIK3 T163 mutation, such as a mutation affecting the activation of SIK3. In preferred embodiments a SIK3 protein of the invention is not a SIK1 (synonyms: SIK and SNF1LK) protein and/or is not a SIK2 (synonyms: QIK, KIAA0781 and SNF1LK2) protein. The amino acid sequence of human SIK1 (UniProt: P57059; entry version 168 of 15-Mar-2017) and human SIK2 (UniProt: Q9H0K1; entry version 153 of 15-Mar-2017) are incorporated herein by reference. The term SIK3 can mean, as applicable to the context (if not more specifically indicated), a SIK3 protein (such as one described above) or an mRNA molecule encoding such a SIK3 protein. The analogous meaning with respect of "SIK1" and "SIK2" is to be understood.

A compound being an "inhibitor of SIK3" (or "SIK3 inhibitor") is any moiety that inhibits SIK3, which can mean inhibition of the activity of SIK3, especially of protein of SIK3, and in particular of phosphorylated SIK3. A SIK3 inhibitor may impair (eg, induces a decrease or reduction in) the efficiency, effectiveness, amount or rate of one or more activities of SIK3, such as one or more of those activities described herein, for example, the activity of SIK3 to phosphorylate class II (eg IIa) HDACs (eg HDAC4) and/or to sensitise a cell involved with a proliferative disorder to a cell-mediated immune response.

In **a first embodiment,** and as may be further described, defined, claimed or otherwise disclosed herein, the present invention provides as a SIK3 inhibitor **a compound** selected from the group consisting of a kinase inhibitor of the formula: 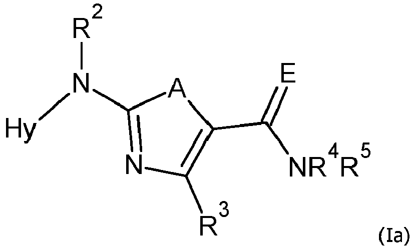 and solvates, salts, N-oxides, complexes, polymorphs, crystalline forms, racemic mixtures, diastereomers, enantiomers, tautomers, conformers, isotopically labeled forms, prodrugs, and combinations thereof; wherein:
**Hy** is a heteroaryl or heterocyclyl which is optionally substituted with one or more independently selected R^{1e};
**each R^{1e}** is independently selected from the group consisting of R^{1a}, R^{1b}, R^{1c} and R^{1d};
**each of R^{1a} and R^{1d}** is independently selected from the group consisting of H, alkyl, alkenyl, alkynyl, cycloalkyl, aryl, heterocyclyl, heteroaryl, halogen, -CN, azido, -NO₂, -OR¹¹, -N(R¹²)(R¹³), -N(R¹¹)(OR¹¹), -S(O)₀₋₂R¹¹, -S(O)₁₋₂OR¹¹, -OS(O)₁₋₂R¹¹, -OS(O)₁₋₂OR¹¹, -S(O)₁₋₂N(R¹²)(R¹³), -OS(O)₁₋₂N(R¹²)(R¹³), -N(R¹¹)S(O)₁₋₂R¹¹, -NR¹¹S(O)₁₋₂OR¹¹, -NR¹¹S(O)₁₋₂N(R¹²)(R¹³), -P(O)(OR¹¹)₂, -OP(O)(OR¹¹)₂, -C(=X)R¹¹, -C(=X)XR¹¹, -XC(=X)R¹¹, and -XC(=X)XR¹¹, wherein each of the alkyl, alkenyl, alkynyl, cycloalkyl, aryl, heterocyclyl, and heteroaryl groups is optionally substituted with one or more independently selected R³⁰;
**each of R^{1b} and R^{1c}** is independently selected from the group consisting of H, C₁₋₆ alkyl, C₂₋₆ alkenyl, C₂₋₆ alkynyl, C₃₋₇ cycloalkyl, C₆₋₁₀ aryl, 3- to 7-membered heteroaryl, 3- to 7-membered heterocyclyl, -O(CH₂)₀₋₂(C₃₋₇ cycloalkyl), -O(CH₂)₀₋₂(C₆₋₁₀ aryl), -O(CH₂)₀₋₂(3- to 7-membered heteroaryl), -O(CH₂)₀₋₂(3- to 7-membered heterocyclyl), -NH(CH₂)₀₋₂(C₃₋₇ cycloalkyl), -NH(CH₂)₀₋₂(C₆₋₁₀ aryl), -NH(CH₂)₀₋₂(3- to 7-membered heteroaryl), -NH(CH₂)₀₋₂(3- to 7-membered heterocyclyl), halogen, -CF₃, -CN, azido, -NO₂, -OH, -O(C₁₋₆ alkyl), -OCF₃, -S(C₁₋₆ alkyl), -NH₂, -NH(C₁₋₆ alkyl), -N(C₁₋₆ alkyl)₂, -NHS(O)₂(C₁₋₆ alkyl), -S(O)₂NH_{2-z}(C₁₋₆ alkyl)_{z}, -C(=O)(C₁₋₆ alkyl), -C(=O)OH, -C(=O)O(C₁₋₆ alkyl), -C(=O)NH_{2-z}(C₁₋₆ alkyl)_{z}, -NHC(=O)(C₁₋₆ alkyl), -NHC(=NH)NH_{z-2}(C₁₋₆ alkyl)_{z}, and -N(C₁₋₆ alkyl)C(=NH)NH_{2-z}(C₁₋₆ alkyl)_{z}, wherein z is 0, 1, or 2 and each of the C₁₋₆ alkyl, C₂₋₆ alkenyl, C₂₋₆ alkynyl, C₃₋₇ cycloalkyl, C₆₋₁₀ aryl, 3- to 7-membered heteroaryl, and 3- to 7-membered heterocyclyl groups is optionally substituted with one, two, or three moieties independently selected from the group consisting of -OH, methyl, ethyl, -OCH₃, -SCH₃, and -NH_{2-z}(CH₃)_{z};
**R²** is H;
**R³** is selected from the group consisting of H, alkyl, alkenyl, alkynyl, cycloalkyl, aryl, heterocyclyl, heteroaryl, halogen, -CN, azido, -NO₂, -OR¹¹, -N(R¹²)(R¹³), -N(R¹¹(OR¹¹), -S(O)₀₋₂R¹¹, -S(O)₁₋₂OR¹¹, -OS(O)₁₋₂R¹¹, -OS(O)₁₋₂OR¹¹, -S(O)₁₋₂N(R¹²)(R¹³), -OS(O)₁₋₂N(R¹²)(R¹³), -N(R¹¹)S(O)₁₋₂R¹¹, -NR¹¹S(O)₁₋₂OR¹¹, -NR¹¹S(O)₁₋₂N(R¹²)(R¹³), -P(O)(OR¹¹)₂, -OP(O)(OR¹¹)₂, -C(=X)R¹¹, -C(=X)XR¹¹, -XC(=X)R¹¹, and -XC(=X)XR¹¹, wherein each of the alkyl, alkenyl, alkynyl, cycloalkyl, aryl, heterocyclyl, and heteroaryl groups is optionally substituted with one or more independently selected R³⁰;
**R⁴** is H;
**R⁵** is -L-R⁶;
**L** is selected from the group consisting of a bond, C₁₋₆ alkylene, C₂₋₆ alkenylene, C₂₋₆ alkynylene, and -(CH₂)ₘ-[Y-(CH₂)ₙ]ₒ-, wherein m is an integer between 1 and 6, n is an integer between 0 and 3, o is an integer between 1 and 3, wherein if n is 0 then o is 1; Y is independently selected from O, S, and -N(R¹³)-; and each of the C₁₋₆ alkylene, C₂₋₆ alkenylene, C₂₋₆ alkynylene, -(CH₂)ₘ-, and -(CH₂)ₙ- groups is optionally substituted with one or two independently selected R³⁰;
**R⁶** is a 5-membered monocyclic heteroaryl which contains at least one S ring atom and which is substituted with one or more independently selected R⁷;
**R⁷** is independently selected from the group consisting of alkyl, alkenyl, alkynyl, cycloalkyl, aryl, heterocyclyl, heteroaryl, halogen, -CN, azido, -NO₂, -OR¹¹, -N(R¹²)(R¹³), -N(R¹¹)(OR¹¹), -S(O)₀₋₂R¹¹, -S(O)₁₋₂OR¹¹, -OS(O)₁₋₂R¹¹,-OS(O)₁₋₂OR¹¹,
   -S(O)₁₋₂N(R¹²)(R¹³), -OS(O)₁₋₂N(R¹²)(R¹³), -N(R¹¹)S(O)₁₋₂R¹¹, -NR¹¹S(O)₁₋₂OR¹¹, -NR¹¹S(O)₁₋₂N(R¹²)(R¹³), -P(O)(OR¹¹)₂, -OP(O)(OR¹¹)₂, -C(=X)R¹¹, -C(=X)XR¹¹, -XC(=X)R¹¹, and -XC(=X)XR¹¹, wherein each of the alkyl, alkenyl, alkynyl, cycloalkyl, aryl, heterocyclyl, and heteroaryl groups is optionally substituted with one or more independently selected R³⁰, wherein at least one of R⁷ is F and/or at least one of R⁷ is substituted with one or more F atoms;
**A** is selected from the group consisting of S, O, NR⁸, and C(R⁹)₂;
**R⁸** is selected from the group consisting of H, alkyl, alkenyl, alkynyl, cycloalkyl, aryl, heterocyclyl, and heteroaryl, wherein each of the alkyl, alkenyl, alkynyl, cycloalkyl, aryl, heterocyclyl, and heteroaryl groups is optionally substituted with one or more independently selected R³⁰;
**R⁹** is independently selected from the group consisting of alkyl, alkenyl, alkynyl, cycloalkyl, aryl, heterocyclyl, heteroaryl, halogen, -CN, azido, -NO₂, -OR¹¹, -N(R¹²)(R¹³), -S(O)₀₋₂R¹¹, -S(O)₁₋₂OR¹¹, -OS(O)₁₋₂R¹¹, -OS(O)₁₋₂OR¹¹, -S(O)₁₋₂N(R¹²)(R¹³), -OS(O)₁₋₂N(R¹²)(R¹³), -N(R¹¹)S(O)₁₋₂R¹¹, -NR¹¹S(O)₁₋₂OR¹¹, -NR¹¹S(O)₁₋₂N(R¹²)(R¹³), -C(=X)R¹¹, -C(=X)XR¹¹, -XC(=X)R¹¹, and -XC(=X)XR¹¹, wherein each of the alkyl, alkenyl, alkynyl, cycloalkyl, aryl, heterocyclyl, and heteroaryl groups is optionally substituted with one or more independently selected R³⁰;
**X** is independently selected from the group consisting of O, S, and N(R¹⁴);
**E** is O or S;
**R¹¹** is independently selected from the group consisting of H, alkyl, alkenyl, alkynyl, cycloalkyl, aryl, heteroaryl, and heterocyclyl, wherein each of the alkyl, alkenyl, alkynyl, cycloalkyl, aryl, heteroaryl, and heterocyclyl groups is optionally substituted with one or more independently selected R³⁰;
**each of R¹² and R¹³** is independently selected from the group consisting of H, alkyl, alkenyl, alkynyl, cycloalkyl, aryl, heteroaryl, and heterocyclyl, or R¹² and R¹³ may join together with the nitrogen atom to which they are attached to form the group -N=CR¹⁵R¹⁶, wherein each of the alkyl, alkenyl, alkynyl, cycloalkyl, aryl, heteroaryl, and heterocyclyl groups is optionally substituted with one or more independently selected R³⁰;
**R¹⁴** is independently selected from the group consisting of H, alkyl, alkenyl, alkynyl, cycloalkyl, aryl, heteroaryl, heterocyclyl, and -OR¹¹, wherein each of the alkyl, alkenyl, alkynyl, cycloalkyl, aryl, heteroaryl, and heterocyclyl groups is optionally substituted with one or more independently selected R³⁰;
**each of R¹⁵ and R¹⁶** is independently selected from the group consisting of H, alkyl, alkenyl, alkynyl, cycloalkyl, aryl, heteroaryl, heterocyclyl, and -NH_{y}R²⁰_{2-y}, or R¹⁵ and R¹⁶ may join together with the atom to which they are attached to form a ring which is optionally substituted with one or more independently selected R³⁰, wherein each of the alkyl, alkenyl, alkynyl, cycloalkyl, aryl, heteroaryl, and heterocyclyl groups is optionally substituted with one or more independently selected R³⁰;
**y** is an integer from 0 to 2;
**R²⁰** is independently selected from the group consisting of alkyl, alkenyl, alkynyl, cycloalkyl, aryl, heteroaryl, and heterocyclyl, wherein each of the alkyl, alkenyl, alkynyl, cycloalkyl, aryl, heteroaryl, and heterocyclyl groups is optionally substituted with one or more independently selected R³⁰; and
**R³⁰** is a 1^{st} level substituent and is, in each case, independently selected from the group consisting of alkyl, alkenyl, alkynyl, aryl, heteroaryl, cycloalkyl, heterocyclyl, halogen, -CN, azido, -NO₂, -OR⁷¹, -N(R⁷²)(R⁷³), -S(O)₀₋₂R⁷¹, -S(O)₁₋₂OR⁷¹, -OS(O)₁₋₂R⁷¹, -OS(O)₁₋₂OR⁷¹, -S(O)₁₋₂N(R⁷²)(R⁷³), -OS(O)₁₋₂N(R⁷²)(R⁷³), -N(R⁷¹)S(O)₁₋₂R⁷¹, -NR⁷¹S(O)₁₋₂OR⁷¹, -NR⁷¹S(O)₁₋₂N(R⁷²)(R⁷³), -OP(O)(OR⁷¹)₂, -C(=X¹)R⁷¹, -C(=X¹)X¹R⁷¹, -X¹C(=X¹)R⁷¹, and -X¹C(=X¹)X¹R⁷¹, and/or any two R³⁰ which are bound to the same carbon atom of a cycloalkyl or heterocyclyl group may join together to form =X¹, wherein each of the alkyl, alkenyl, alkynyl, aryl, heteroaryl, cycloalkyl, and heterocyclyl groups being a 1^{st} level substituent is optionally substituted by one or more 2^{nd} level substituents, wherein said 2^{nd} level substituent is, in each case, independently selected from the group consisting of C₁₋₆ alkyl, C₂₋₆ alkenyl, C₂₋₆ alkynyl, 3- to 14-membered aryl, 3- to 14-membered heteroaryl, 3- to 14-membered cycloalkyl, 3- to 14-membered heterocyclyl, halogen, -CF₃, -CN, azido, -NO₂, -OR⁸¹, -N(R⁸²)(R⁸³), -S(O)₀₋₂R⁸¹, -S(O)₁₋₂OR⁸¹, -OS(O)₁₋₂R⁸¹, -OS(O)₁₋₂OR⁸¹, -S(O)₁₋₂N(R⁸²)(R⁸³), -OS(O)₁₋₂N(R⁸²)(R⁸³), -N(R⁸¹)S(O)₁₋₂R⁸¹, -NR⁸¹S(O)₁₋₂OR⁸¹, -NR⁸¹S(O)₁₋₂N(R⁸²)(R⁸³), -OP(O)(OR⁸¹)₂, -C(=X²)R⁸¹, -C(=X²)X²R⁸¹, -X²C(=X²)R⁸¹, and -X²C(=X²)X²R⁸¹, and/or any two 2^{nd} level substituents which are bound to the same carbon atom of a cycloalkyl or heterocyclyl group being a 1^{st} level substituent may join together to form =X², wherein each of the C₁₋₆ alkyl, C₂₋₆ alkenyl, C₂₋₆ alkynyl, 3- to 14-membered aryl, 3- to 14-membered heteroaryl, 3- to 14-membered cycloalkyl, 3- to 14-membered heterocyclyl groups being a 2^{nd} level substituent is optionally substituted with one or more 3^{rd} level substituents, wherein said 3^{rd} level substituent is, in each case, independently selected from the group consisting of C₁₋₃ alkyl, halogen, -CF₃, -CN, azido, -NO₂, -OH, -O(C₁₋₃ alkyl), -OCF₃, -S(C₁₋₃ alkyl), -NH₂, -NH(C₁₋₃ alkyl), -N(C₁₋₃ alkyl)₂, -NHS(O)₂(C₁₋₃ alkyl), -S(O)₂NH_{2-z}(C₁₋₃ alkyl)_{z}, -C(=O)OH, -C(=O)o(C₁₋₃ alkyl), -C(=O)NH_{2-z}(C₁₋₃ alkyl)_{z}, -NHC(=O)(C₁₋₃ alkyl), -NHC(=NH)NH_{z-2}(C₁₋₃ alkyl)_{z}, and -N(C₁₋₃ alkyl)C(=NH)NH_{2-z}(C₁₋₃ alkyl)_{z}, wherein each z is independently 0, 1, or 2 and each C₁₋₃ alkyl is independently methyl, ethyl, propyl or isopropyl, and/or any two 3^{rd} level substituents which are bound to the same carbon atom of a 3- to 14-membered cycloalkyl or heterocyclyl group being a 2^{nd} level substituent may join together to form =O, =S, =NH, or =N(C₁₋₃ alkyl);
   wherein
**each of R⁷¹, R⁷², and R⁷³** is independently selected from the group consisting of H, C₁₋₆ alkyl, C₂₋₆ alkenyl, C₂₋₆ alkynyl, 3- to 7-membered cycloalkyl, 5- or 6-membered aryl, 5- or 6-membered heteroaryl, and 3- to 7-membered heterocyclyl, wherein each of the C₁₋₆ alkyl, C₂₋₆ alkenyl, C₂₋₆ alkynyl, 3- to 7-membered cycloalkyl, 5- or 6-membered aryl, 5- or 6-membered heteroaryl, and 3- to 7-membered heterocyclyl groups is optionally substituted with one, two or three substituents independently selected from the group consisting of C₁₋₃ alkyl, halogen, -CF₃, -CN, azido, -NO₂, -OH, -O(C₁₋₃ alkyl), -OCF₃, =O, -S(C₁₋₃ alkyl), -NH₂, -NH(C₁₋₃ alkyl), -N(C₁₋₃ alkyl)₂, -NHS(O)₂(C₁₋₃ alkyl), -S(O)₂NH_{2-z}(C₁₋₃ alkyl)_{z}, -C(=O)(C₁₋₃ alkyl), -C(=O)OH, -C(=O)O(C₁₋₃ alkyl), -C(=O)NH_{2-z}(C₁₋₃ alkyl)_{z}, -NHC(=O)(C₁₋₃ alkyl), -NHC(=NH)NH_{z-2}(C₁₋₃ alkyl)_{z}, and -N(C₁₋₃ alkyl)C(=NH)NH_{2-z}(C₁₋₃ alkyl)_{z}, wherein each z is independently 0, 1, or 2 and each C₁₋₃ alkyl is independently methyl, ethyl, propyl or isopropyl;
**each of R⁸¹, R⁸², and R⁸³** is independently selected from the group consisting of H, C₁₋₄ alkyl, C₂₋₄ alkenyl, C₂₋₄ alkynyl, 3- to 6-membered cycloalkyl, 5- or 6-membered aryl, 5- or 6-membered heteroaryl, and 3- to 6-membered heterocyclyl, wherein each of the C₁₋₄ alkyl, C₂₋₄ alkenyl, C₂₋₄ alkynyl, 3- to 6-membered cycloalkyl, 5- or 6-membered aryl, 5- or 6-membered heteroaryl, and 3- to 6-membered heterocyclyl groups is optionally substituted with one, two or three substituents independently selected from the group consisting of C₁₋₃ alkyl, halogen, -CF₃, -CN, azido, -NO₂, -OH, -O(C₁₋₃ alkyl), -OCF₃, =O, -S(C₁₋₃ alkyl), -NH₂, -NH(C₁₋₃ alkyl), -N(C₁₋₃ alkyl)₂, -NHS(O)₂(C₁₋₃ alkyl), -S(O)₂NH_{2-z}(C₁₋₃ alkyl)_{z}, -C(=O)(C₁₋₃ alkyl), -C(=O)OH, -C(=O)O(C₁₋₃ alkyl), -C(=O)NH_{2-z}(C₁₋₃ alkyl)_{z}, -NHC(=O)(C₁₋₃ alkyl), -NHC(=NH)NH_{z-2}(C₁₋₃ alkyl)_{z}, and -N(C₁₋₃ alkyl)C(=NH)NH_{2-z}(C₁₋₃ alkyl)_{z}, wherein each z is independently 0, 1, or 2 and **each C₁₋₃** alkyl is independently methyl, ethyl, propyl or isopropyl; and
**each of X¹ and X²** is independently selected from O, S, and N(R⁸⁴), wherein R⁸⁴ is H or C₁₋₃ alkyl.

Such compounds are which are kinase inhibitors are in detail defined and exemplified in international patent publication WO 2021/214117 which shall for the purpose of the definition of SIK3 inhibitors of the invention be incorporated herein in its entirety.

A selection of SIK3 inhibitor compounds, including those which have been synthesized and tested, within the scope of, or for use within the methods of, the disclosure of WO 2021/214117 - is listed in the following Table A.

**Table A: Kinase inhibitors of formula (Ia).**

| Compound Number | Structure | Name |
|---|---|---|
| E1 | 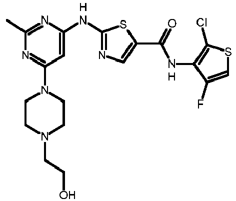 | *N*-(2-chloro-4-fluorothiophen-3-yl)-2-((6-(4-(2-hydroxyethyl)piperazin-1-yl)-2-methylpyrimidin-4-yl)amino)thiazole-5-carboxamide |
| E2 | 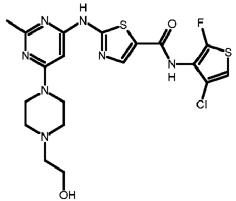 | *N*-(4-chloro-2-fluorothiophen-3-yl)-2-((6-(4-(2-hydroxyethyl)piperazin-1-yl)-2-methylpyrimidin-4-yl)amino)thiazole-5-carboxamide |
| E3 | 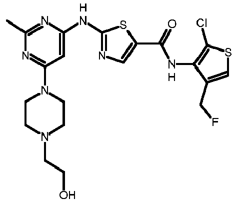 | *N*-(2-chloro-4-(fluoromethyl)thiophen-3-yl)-2-((6-(4-(2-hydroxyethyl)piperazin-1-yl)-2-methylpyrimidin-4-yl)amino)thiazole-5-carboxamide |
| E4 | 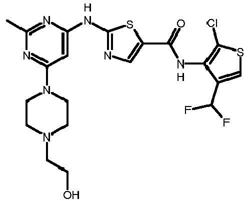 | *N*-(2-chloro-4-(difluoromethyl)thiophen-3-yl)-2-((6-(4-(2-hydroxyethyl)piperazin-1-yl)-2-methylpyrimidin-4-yl)amino)thiazole-5-carboxamide |
| E5 | 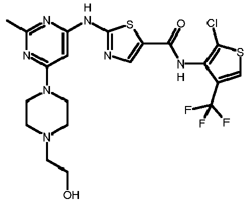 | *N*-(2-chloro-4-(trifluoromethyl)thiophen-3-yl)-2-((6-(4-(2-hydroxyethyl)piperazin-1-yl)-2-methylpyrimidin-4-yl)amino)thiazole-5-carboxamide |
| E6 | 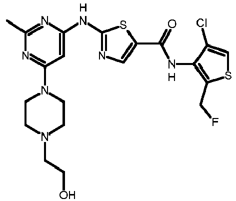 | *N*-(4-chloro-2-(fluoromethyl)thiophen-3-yl)-2-((6-(4-(2-hydroxyethyl)piperazin-1-yl)-2-methylpyrimidin-4-yl)amino)thiazole-5-carboxamide |
| E7 | 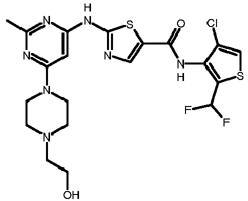 | *N*-(4-chloro-2-(difluoromethyl)thiophen-3-yl)-2-((6-(4-(2-hydroxyethyl)piperazin-1-yl)-2-methylpyrimidin-4-yl)amino)thiazole-5-carboxamide |
| E8 | 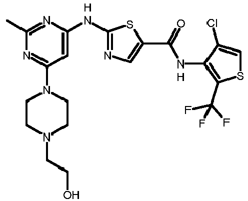 | *N*-(4-chloro-2-(trifluoromethyl)thiophen-3-yl)-2-((6-(4-(2-hydroxyethyl)piperazin-1-yl)-2-methylpyrimidin-4-yl)amino)thiazole-5-carboxamide |
| E9 | 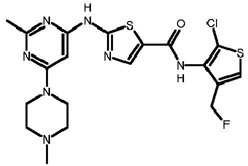 | *N*-(2-chloro-4-(fluoromethyl)thiophen-3-yl)-2-((2-methyl-6-(4-methylpiperazin-1-yl)pyrimidin-4-yl)amino)thiazole-5-carboxamide |
| E10 | 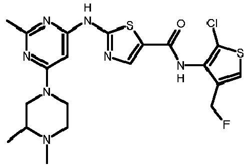 | *N*-(2-chloro-4-(fluoromethyl)thiophen-3-yl)-2-((6-(3,4-dimethylpiperazin-1-yl)-2-methylpyrimidin-4-yl)amino)thiazole-5-carboxamide |
| E11 | 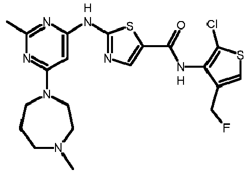 | *N*-(2-chloro-4-(fluoromethyl)thiophen-3-yl)-2-((2-methyl-6-(4-methyl-1,4-diazepan-1-yl)pyrimidin-4-yl)amino)thiazole-5-carboxamide |
| E12 | 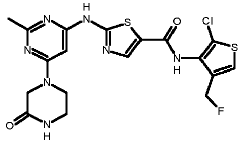 | *N*-(2-chloro-4-(fluoromethyl)thiophen-3-yl)-2-((2-methyl-6-(3-oxopiperazin-1-yl)pyrimidin-4-yl)amino)thiazole-5-carboxamide |
| E13 | 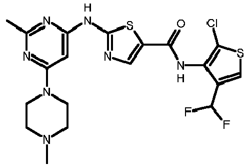 | *N*-(2-chloro-4-(difluoromethyl)thiophen-3-yl)-2-((6-(3,4-dimethylpiperazin-1-yl)-2-methyl-pyrimidin-4-yl)amino)thiazole-5-carboxamide |
| E14 | 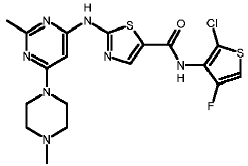 | *N*-(2-chloro-4-fluorothiophen-3-yl)-2-((2-methyl-6-(4-methylpiperazin-1-yl)pyrimidin-4-yl)amino)thiazole-5-carboxamide |
| E15 | 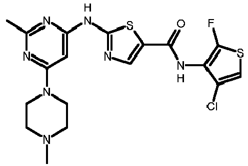 | *N*-(4-chloro-2-fluorothiophen-3-yl)-2-((2-methyl-6-(4-methylpiperazin-1-yl)pyrimidin-4-yl)amino)thiazole-5-carboxamide |
| E16 | 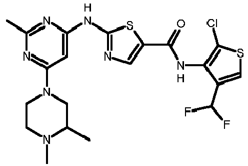 | *N*-(2-chloro-4-(difluoromethyl)thiophen-3-yl)-2-((2-methyl-6-(3,4-dimethylpiperazin-1-yl)pyrimidin-4-yl)amino)thiazole-5-carboxamide |

In particular embodiments, the compound of the invention is selected from the group consisting of E4, E9, E10, and E16; and also their solvates, salts, N-oxides, complexes, polymorphs, crystalline forms, racemic mixtures, diastereomers, enantiomers, tautomers, conformers, isotopically labelled forms, prodrugs, and combinations thereof.

In certain embodiments, the compound of the invention is E9, or a solvate, salt, N-oxide, complex, polymorph, crystalline form, tautomer, conformer, isotopically labelled form, prodrug, or combination thereof.

In another certain embodiment, the compound of the invention is E4, or a solvate, salt, N-oxide, complex, polymorph, crystalline form, tautomer, conformer, isotopically labelled form, prodrug, or combination thereof.

In another certain embodiment, the compound of the invention is E10, or a solvate, salt, N-oxide, complex, polymorph, crystalline form, tautomer, conformer, isotopically labelled form, prodrug, or combination thereof.

In another certain embodiment, the compound of the invention is E16, or a solvate, salt, N-oxide, complex, polymorph, crystalline form, racemic mixture, diastereomer, enantiomer, tautomer, conformer, isotopically labelled form, prodrug, or combination thereof.

In certain embodiments, the invention may relate to a solvate, salt, N-oxide, complex, racemic mixture, diastereomer, enantiomer, tautomer, conformer, isotopically labeled form, prodrug, or combination thereof, of any of the compounds of the invention or of any of the compounds used in the invention; such as a solvate, salt, complex, racemic mixture, diastereomer, enantiomer, tautomer, conformer, isotopically labeled form, or combination thereof, of such compound.

The SIK3 inhibitor compounds of the invention (and/or the compounds used in the invention) may be in a prodrug form. Prodrugs of the compounds of the invention (or of the compounds used in the invention) are those compounds that upon administration to an individual undergo chemical conversion under physiological conditions to provide the compounds of the invention. Additionally, prodrugs can be converted to the compounds of the invention (or of the compounds used in the invention) by chemical or biochemical methods in an *ex vivo* environment. For example, prodrugs can be slowly converted to the compounds of the invention (or to the compounds used in the invention) when, for example, placed in a transdermal patch reservoir with a suitable enzyme or chemical reagent. Exemplary prodrugs are esters (using an alcohol or a carboxy group contained in the kinase inhibitor of the invention (or in the compounds used in the invention)) or amides (using an amino or a carboxy group contained in the kinase inhibitor of the invention (or in the compounds used in the invention)) which are hydrolyzable *in vivo.* Specifically, any amino group which is contained in the kinase inhibitor of the invention (or in the compounds used in the invention) and which bears at least one hydrogen atom can be converted into a prodrug form.

In another embodiment, the compounds of the invention exhibit one or more pharmacological properties that are different to those of dasatinib, of compound B3 (WO 2018/193084), and/or of compound C7 (PCT/EP2019/078751).

Other than the above small molecular kinase inhibitors, the invention shall also include other strategies to interfere with the cellular function of SIK3. Other compounds are for example known from WO/2021/219731, WO/2019/202160 and WO/2018/193084.

A compound being an "inhibitor of SIK3" (or "SIK3 inhibitor") is any moiety that inhibits SIK3, which can mean inhibition of the activity of SIK3, especially of protein of SIK3, and in particular of phosphorylated SIK3. A SIK3 inhibitor may impair (eg, induces a decrease or reduction in) the efficiency, effectiveness, amount or rate of one or more activities of SIK3, such as one or more of those activities described herein, for example, the activity of SIK3 to phosphorylate class II (eg Ila) HDACs (eg HDAC4) and/or to sensitise a cell involved with a proliferative disorder to a cell-mediated immune response. Such a SIK3 inhibiting moiety can act directly, for example, by binding to SIK3 and decreasing the amount or rate of one or more of the properties of SIK3 such as its function, in particular its ability to act as a kinase (eg to phosphorylate HDAC4), for example by reducing the activity of phosphorylated SIK3 in the cell.

In certain embodiments of these combination aspects, the inhibitor or antagonist of said SIK3 kinase or variant is, preferably, an inhibitory or antagonistic small molecule as described herein. In other embodiments of these combination aspects, said inhibitor or antagonist of said SIK3 or variant, is a compound selected from a polypeptide, peptide, glycoprotein, a peptidomimetic, an antibody or antibody-like molecule; a nucleic acid such as a DNA or RNA, for example an antisense DNA or RNA, a ribozyme, an RNA or DNA aptamer, siRNA, shRNA and the like, including variants or derivatives thereof such as a peptide nucleic acid (PNA); a targeted gene editing construct, such as a CRISPR/Cas9 construct, a carbohydrate such as a polysaccharide or oligosaccharide and the like, including variants or derivatives thereof; a lipid such as a fatty acid and the like, including variants or derivatives thereof; or a small organic molecules including but not limited to small molecule ligands, small cell-permeable molecules, and peptidomimetic compounds.

### Pharmaceutical compositions

The compounds described in the present invention (in particular those SIK3 inhibitor compounds above particularly those given in Table A) or the compounds used in the present invention are preferably administered to a patient in need thereof via a pharmaceutical composition. Thus, in **a second aspect,** the present invention provides **a pharmaceutical composition** comprising a kinase inhibitor as specified above under the heading "Compounds" (e.g., a SIK3 kinase inhibitor having the general formula (Ia), complex, polymorph, crystalline form, racemic mixture, diastereomer, enantiomer, tautomer, conformer, isotopically labeled form, prodrug, or combination thereof and optionally one or more pharmaceutically acceptable excipients.

Thus, in one embodiment the pharmaceutical composition comprises a kinase inhibitor as specified above under the heading "Compounds" (in particular a compound of the first aspect of the invention) and one or more pharmaceutically acceptable excipients. Furthermore, the pharmaceutical composition may further comprise one or more additional therapeutic agents. Thus, in particular embodiments, the pharmaceutical composition comprises (i) a kinase inhibitor as specified above under the heading "Compounds" (in particular a compound of the first aspect of the invention) and one or more additional therapeutic agents; or (ii) a kinase inhibitor as specified above under the heading "Compounds" (in particular a compound of the first aspect of the invention), one or more additional therapeutic agents, and one or more pharmaceutically acceptable excipients.

The term "pharmaceutically acceptable" refers to the non-toxicity of a material which does not interact with the (e.g., therapeutic) action of the active component (e.g., a kinase inhibitor of the invention (or a compound used in the invention), either alone or in combination with one or more additional therapeutic agents) of the pharmaceutical composition.

The pharmaceutical composition may be administered to an individual by any route, such as enterally or parenterally.

The expressions "enteral administration" and "administered enterally" as used herein mean that the drug administered is taken up by the stomach and/or the intestine. Examples of enteral administration include oral and rectal administration. The expressions "parenteral administration" and "administered parenterally" as used herein mean modes of administration other than enteral administration, usually by injection or topical application, and include, without limitation, intravenous, intramuscular, intraarterial, intrathecal, intracapsular, intraosseous, intraorbital, intracardiac, intradermal, intraperitoneal, transtracheal, subcutaneous, subcuticular, intraarticular, subcapsular, intracerebral, intracerebroventricular, subarachnoid, intraspinal, epidural and intrasternal administration (such as by injection and/or infusion) as well as topical administration (e.g., epicutaneous, inhalational, or through mucous membranes (such as buccal, sublingual or vaginal)).

Dosage forms for topical and/or transdermal administration of a compound described herein may include ointments, pastes, creams, lotions, gels, powders, solutions, sprays, inhalants, and/or patches. Generally, the active ingredient is admixed under sterile conditions with a pharmaceutically acceptable excipient such as one or more pharmaceutical carriers) and/or any needed preservatives and/or buffers as can be required. Additionally, the present disclosure contemplates the use of transdermal patches, which often have the added advantage of providing controlled delivery of an active ingredient to the body. Such dosage forms can be prepared, for example, by dissolving and/or dispensing the active ingredient in the proper medium. Alternatively, or additionally, the rate can be controlled by either providing a rate controlling membrane and/or by dispersing the active ingredient in a polymer matrix and/or gel.

Suitable devices for use in delivering intradermal pharmaceutical compositions described herein include short needle devices. Intradermal compositions can be administered by devices which limit the effective penetration length of a needle into the skin. Alternatively, or additionally, conventional syringes can be used in the classical mantoux method of intradermal administration. Jet injection devices which deliver liquid formulations to the dermis via a liquid jet injector and/or via a needle which pierces the stratum corneum and produces a jet which reaches the dermis are suitable. Ballistic powder/particle delivery devices which use compressed gas to accelerate the compound in powder form through the outer layers of the skin to the dermis are suitable.

Formulations suitable for topical administration include, but are not limited to, liquid and/or semi-liquid preparations such as liniments, lotions, oil-in-water and/or water-in-oil emulsions such as creams, ointments, and/or pastes, and/or solutions and/or suspensions. Topically administrable formulations may, for example, comprise from about 1% to about 10% (w/w) active ingredient, although the concentration of the active ingredient can be as high as the solubility limit of the active ingredient in the solvent. Formulations for topical administration may further comprise one or more of the additional ingredients described herein.

The compounds of the present invention (or the compounds used in the present invention) are generally applied in "pharmaceutically acceptable amounts" and in "pharmaceutically acceptable preparations". Such compositions may contain salts, buffers, preserving agents, carriers and optionally other therapeutic agents. "Pharmaceutically acceptable salts" comprise, for example, acid addition salts which may, for example, be formed by mixing a solution of compounds with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compound carries an acidic moiety, suitable pharmaceutically acceptable salts thereof may include alkali metal salts (e.g., sodium or potassium salts); alkaline earth metal salts (e.g., calcium or magnesium salts); and salts formed with suitable organic ligands (e.g., ammonium, quaternary ammonium and amine cations formed using counteranions such as halide, hydroxide, carboxylate, sulfate, phosphate, nitrate, alkyl sulfonate and aryl sulfonate). Illustrative examples of pharmaceutically acceptable salts include, but are not limited to, acetate, adipate, alginate, arginate, ascorbate, aspartate, benzenesulfonate, benzoate, bicarbonate, bisulfate, bitartrate, borate, bromide, butyrate, calcium edetate, camphorate, camphorsulfonate, camsylate, carbonate, chloride, citrate, clavulanate, cyclopentanepropionate, digluconate, dihydrochloride, dodecylsulfate, edetate, edisylate, estolate, esylate, ethanesulfonate, formate, fumarate, galactate, galacturonate, gluceptate, glucoheptonate, gluconate, glutamate, glycerophosphate, glycolylarsanilate, hemisulfate, heptanoate, hexanoate, hexylresorcinate, hydrabamine, hydrobromide, hydrochloride, hydroiodide, 2-hydroxy-ethanesulfonate, hydroxynaphthoate, iodide, isobutyrate, isothionate, lactate, lactobionate, laurate, lauryl sulfate, malate, maleate, malonate, mandelate, mesylate, methanesulfonate, methylsulfate, mucate, 2-naphthalenesulfonate, napsylate, nicotinate, nitrate, N-methylglucamine ammonium salt, oleate, oxalate, pamoate (embonate), palmitate, pantothenate, pectinate, persulfate, 3-phenylpropionate, phosphate/diphosphate, phthalate, picrate, pivalate, polygalacturonate, propionate, salicylate, stearate, sulfate, suberate, succinate, tannate, tartrate, teoclate, tosylate, triethiodide, undecanoate, valerate, and the like (see, for example, Berge et al., "Pharmaceutical Salts", J. Pharm. Sci., 66, pp. 1-19 (1977)).

The term "excipient" when used herein is intended to indicate all substances in a pharmaceutical composition which are not active ingredients (e.g., which are therapeutically inactive ingredients that do not exhibit any therapeutic effect in the amount/concentration used), such as, e.g., carriers, binders, lubricants, thickeners, surface active agents, preservatives, stabilizers, emulsifiers, buffers, flavoring agents, colorants, or antioxidants.

The compositions described in the present invention may comprise a pharmaceutically acceptable carrier. As used herein, "pharmaceutically acceptable carrier" includes any and all solvents, dispersion media, coatings, isotonic and absorption delaying agents, and the like that are physiologically compatible. The "pharmaceutically acceptable carrier" may be in the form of a solid, semisolid, liquid, or combinations thereof. Preferably, the carrier is suitable for enteral (such as oral) or parenteral administration (such as intravenous, intramuscular, subcutaneous, spinal or epidermal administration (e.g., by injection or infusion)). Depending on the route of administration, the active compound, e.g., the compound of the present invention (or the compound used in the present invention), either alone or in combination with one or more additional therapeutic agents, may be coated in a material to protect the active compound(s) from the action of acids and other natural conditions that may inactivate the active compound.

Examples of suitable aqueous and non-aqueous carriers which may be employed in the pharmaceutical compositions according to the present invention include water (e.g., water for injection), ethanol, polyols (such as glycerol, propylene glycol, polyethylene glycol, and the like), aqueous solutions of a salt, carbohydrate, sugar alcohol, or an amino acid (such as saline or an aqueous amino acid solution), and suitable mixtures and/or buffered forms thereof, vegetable oils (such as olive oil), and injectable organic esters (such as ethyl oleate). Proper fluidity can be maintained, for example, by the use of coating materials, such as lecithin, by the maintenance of the required particle size in the case of dispersions, and by the use of surfactants.

Pharmaceutically acceptable carriers include sterile aqueous solutions or dispersions and sterile powders for the extemporaneous preparation of sterile injectable solutions or dispersions. The use of such media and agents for pharmaceutically active compounds is known in the art. Except insofar as any conventional media or agent is incompatible with the active compound, use thereof in the pharmaceutical compositions according to the present invention is contemplated.

The pharmaceutical composition described herein may comprise, in addition to the kinase inhibitor of the invention and the TRAIL or TRAIL agonist (and/or the compound or composition used in the invention), at least one, e.g., 1, 2, 3, 4, 5, 6, 7 or 8, additional therapeutic agents. According to the present teaching, the at least one additional therapeutic agent may be formulated together with the kinase inhibitor of the invention (and/or with the compound used in the invention) in a single pharmaceutical composition. Alternatively, the pharmaceutical composition may be structured as kit of parts, wherein the kinase inhibitor of the invention (or the compound used in the invention) is provided in a first formulation and the at least one additional therapeutic agent is provided in a second formulation, i.e., a second pharmaceutical composition. The first and the second pharmaceutical compositions may be combined prior to use. In other words, before administering the pharmaceutical composition, a formulation comprising the additional therapeutic agent may be added to the first pharmaceutical composition comprising the kinase inhibitor of the invention (or the compound used in the invention). Alternatively, the present teaching envisages administering the kinase inhibitor of the invention (or the compound used in the invention) formulated in a first pharmaceutical composition and administering the at least one additional therapeutic agent formulated in a second pharmaceutical composition. The pharmaceutical compositions may be administered concomitantly or in succession. For example, the first pharmaceutical composition may be administered at a first point in time and the second pharmaceutical composition may be administered at a second point in time, wherein the points in time may be separated by, for example, 0, or up to 1, 2, 3, 4, 5 or 10 min, up to 1, 2, 3, 4, 5 or 10 hours, up to 1, 2, 3, 4, 5 or 10 days, up to 1, 2, 3, 4, 5 or 10 weeks, up to 1, 2, 3, 4, 5 or 10 months or up to 1, 2, 3, 4, 5 or 10 years.

The compositions may also contain adjuvants such as preservatives, stabilizers, wetting agents, emulsifying agents, pH buffering agents, and dispersing agents. Prevention of the presence of microorganisms may be ensured by sterilization procedures and/or by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Regardless of the route of administration selected, the active compounds, which may be used in a suitable hydrated form, and/or the pharmaceutical compositions according to the present invention, are formulated into pharmaceutically acceptable dosage forms by conventional methods known to those of skill in the art (cf., e.g., Remington, "The Science and Practice of Pharmacy" edited by Allen, Loyd V., Jr., 22nd edition, Pharmaceutical Sciences, September 2012; Ansel et al., "Pharmaceutical Dosage Forms and Drug Delivery Systems", 7th edition, Lippincott Williams & Wilkins Publishers, 1999).

A pharmaceutical composition can be administered by a variety of methods known in the art. As will be appreciated by the skilled artisan, the route and/or mode of administration will vary depending upon the desired results. The pharmaceutical compositions containing one or more active compounds can be prepared with carriers that will protect the one or more active compounds against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Methods for the preparation of such compositions are generally known to those skilled in the art. See, e.g., Sustained and Controlled Release Drug Delivery Systems, J. R. Robinson, ed., Marcel Dekker, Inc., New York, 1978.

To administer a compound of the present invention (or a compound used in the present invention) by certain routes of administration, it may be necessary to coat the compound with, or co-administer the compound with, a material to prevent its inactivation. For example, the compound may be administered to an individual in an appropriate carrier, for example, liposomes, or a diluent. Pharmaceutically acceptable diluents include saline and aqueous buffer solutions. Liposomes include water-in-oil-in-water CGF emulsions as well as conventional liposomes (Strejan et al., J. Neuroimmunol. 7: 27(1984)).

Pharmaceutical compositions typically are sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of the injectable compositions can be brought about by including in the composition an agent that delays absorption, for example, monostearate salts and gelatin.

An injectable composition should be sterile and fluid to the extent that the composition is deliverable by syringe. In addition to water, the carrier can be an isotonic buffered saline solution, ethanol, polyol (for example, glycerol, propylene glycol, and liquid polyethylene glycol, and the like), and suitable mixtures thereof. Sterile injectable solutions can be prepared by incorporating the active compound in the required amount in an appropriate solvent with one or a combination of ingredients enumerated above, as required, followed by sterilization microfiltration.

Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the individuals to be treated; each unit contains a predetermined quantity of active compound calculated to produce the desired therapeutic effect in association with the required pharmaceutical carrier. The specification for the dosage unit forms used according to the present invention are dictated by and directly dependent on (a) the unique characteristics of the active compound and the particular therapeutic effect to be achieved, and (b) the limitations inherent in the art of compounding such an active compound for the treatment of sensitivity in individuals.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

For the therapeutic/pharmaceutical formulations, compositions according to the present invention include those suitable for enteral administration (such as oral or rectal) or parenteral administration (such as nasal, topical (including vaginal, buccal and sublingual)). The compositions may conveniently be presented in unit dosage form and may be prepared by any methods known in the art of pharmacy. The amount of active ingredient (in particular, the amount of a compound according to the present invention) which can be combined with a carrier material to produce a pharmaceutical composition (such as a single dosage form) will vary depending upon the individual being treated, and the particular mode of administration. The amount of active ingredient which can be combined with a carrier material to produce a single dosage form will generally be that amount of the composition which produces a therapeutic effect.

Generally, out of 100% (for the pharmaceutical formulations/compositions), the amount of active ingredient (in particular, the amount of the compound according to the present invention (or of the compound used in the present invention), optionally together with other therapeutically active agents, if present in the pharmaceutical formulations/compositions) will range from about 0.01% to about 99%, preferably from about 0.1% to about 70%, most preferably from about 1% to about 30%, wherein the reminder is preferably composed of the one or more pharmaceutically acceptable excipients.

The amount of active ingredient, e.g., a compound according to the present invention (or a compound used in the present invention), in a unit dosage form and/or when administered to an individual or used in therapy, may range from about 0.1 mg to about 1000 mg (for example, from about 1 mg to about 500 mg, such as from about 10 mg to about 200 mg) per unit, administration or therapy. In certain embodiments, a suitable amount of such active ingredient may be calculated using the mass or body surface area of the individual, including amounts of between about 1 mg/kg and 10 mg/kg (such as between about 2 mg/kg and 5 mg/kg), or between about 1 mg/m² and about 400 mg/m² (such as between about 3 mg/m² and about 350 mg/m² or between about 10 mg/m² and about 200 mg/m²).

Actual dosage levels of the active ingredients in the pharmaceutical compositions according to the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

A physician or veterinarian having ordinary skill in the art can readily determine and prescribe the (e.g., therapeutically) effective amount of the pharmaceutical composition required. For example, the physician or veterinarian could start with doses of the compounds according to the present invention (or of the compounds used in the present invention) at levels lower than that required in order to achieve the desired therapeutic effect and gradually increase the dosage until the desired effect is achieved. In general, a suitable daily dose of a composition according to the present invention will be that amount of the compound which is the lowest dose effective to produce a therapeutic effect. Such an effective dose will generally depend upon the factors described above. It is preferred that administration be oral, intravenous, intramuscular, intraperitoneal, or subcutaneous, preferably administered proximal to the site of the target. If desired, the (e.g., therapeutically) effective daily dose of a pharmaceutical composition may be administered as two, three, four, five, six or more sub-doses administered separately at appropriate intervals throughout the day, optionally, in unit dosage forms. While it is possible for a compound according to the present invention (or for the compound used in the present invention) to be administered alone, it is preferable to administer the compound as a pharmaceutical formulation/composition.

For oral administration, the pharmaceutical composition according to the present invention can take the form of, for example, tablets or capsules prepared by conventional means with pharmaceutical acceptable excipients such as binding agents (e.g., pregelatinised maize starch, polyvinylpyrrolidone, hydroxypropyl methylcellulose), fillers (e.g., lactose, microcrystalline cellulose, calcium hydrogen phosphate), lubricants (e.g., magnesium stearate, talc, silica), disintegrants (e.g., potato starch, sodium starch glycolate), or wetting agents (e.g., sodium lauryl sulphate). Liquid preparations for oral administration can be in the form of, for example, solutions, syrups, or suspensions, or can be presented as a dry product for constitution with water or other suitable vehicle before use. Such liquid preparation can be prepared by conventional means with pharmaceutically acceptable additives such as suspending agents (e.g., sorbitol, syrup, cellulose derivatives, hydrogenated edible fats), emulsifying agents (e.g., lecithin, acacia), non-aqueous vehicles (e.g., almond oil, oily esters, ethyl alcohol, fractionated vegetable oils), preservatives (e.g., methyl or propyl-p-hydroxycarbonates, sorbic acids). The preparations can also contain buffer salts, flavouring, coloring and sweetening agents as deemed appropriate. Preparations for oral administration can be suitably formulated to give controlled release of the pharmaceutical composition of the invention.

In one embodiment, the compound is orally administered in a concentration of, for example, at most 100 mg/kg body weight (such as at most 50 mg/kg body weight, at most 40 mg/kg body weight, at most 30 mg/kg body weight, at most 20 mg/kg body weight, at most 10 mg/kg body weight, at most 5 mg/kg body weight, at most 4 mg/kg body weight, at most 3 mg/kg body weight, at most 2 mg/kg body weight, at most 1 mg/kg body weight).

In one embodiment, the compound is parenterally administered (e.g., intravenously, intramuscularly, or subcutaneously), in a concentration of, for example, at most 10 mg/kg body weight (such as at most 5 mg/kg body weight, at most 4 mg/kg body weight, at most 3 mg/kg body weight, at most 2 mg/kg body weight, at most 1 mg/kg body weight, at most 0.5 mg/kg body weight, at most 0.4 mg/kg body weight, at most 0.3 mg/kg body weight, at most 0.2 mg/kg body weight, at most 0.1 mg/kg body weight).

The pharmaceutical composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc.

For administration by inhalation, the pharmaceutical composition according to the present invention is conveniently delivered in the form of an aerosol spray presentation from a pressurized pack or a nebulizer, with the use of a suitable propellant (e.g., dichlorodifluoromethane, trichlorofluoromethane, dichlorotetrafluoroethane, carbon dioxide, nitrogen, or other suitable gas). In the case of a pressurized aerosol, the dosage unit can be determined by providing a valve to deliver a metered amount. Capsules and cartridges of, for example, gelatine, for use in an inhaler or insufflator can be formulated containing a powder mix of the pharmaceutical composition according to the present invention and a suitable powder base such as lactose or starch.

The pharmaceutical composition according to the present invention can be formulated for parenteral administration by injection, for example, by bolus injection or continuous infusion. In one embodiment, the compounds or compositions according to the present invention may be administered by slow continuous infusion over a long period, such as more than 24 hours, in order to reduce toxic side effects. The administration may also be performed by continuous infusion over a period of from 2 to 24 hours, such as of from 2 to 12 hours. Such regimen may be repeated one or more times as necessary, for example, after 6 months or 12 months.

In yet another embodiment, the compounds or compositions according to the present invention are administered by maintenance therapy, such as, e.g., once a week for a period of 6 months or more.

Formulations for injection can be presented in units dosage form (e.g., in phial, in multi-dose container), and with an added preservative. The pharmaceutical composition according to the present invention can take such forms as suspensions, solutions or emulsions in oily or aqueous vehicles, and can contain formulatory agents such as suspending, stabilizing, or dispersing agents. Alternatively, the agent can be in powder form for constitution with a suitable vehicle (e.g., sterile pyrogen-free water) before use. Typically, compositions for intravenous administration are solutions in sterile isotonic aqueous buffer. Where necessary, the composition can also include a solubilizing agent and a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients can be mixed prior to administration.

Compositions according to the present invention which are suitable for vaginal administration also include pessaries, tampons, creams, gels, pastes, foams or spray formulations containing such carriers as are known in the art to be appropriate. Dosage forms for the topical or transdermal administration of compositions according to the present invention include powders, sprays, ointments, pastes, creams, lotions, gels, solutions, patches and inhalants. The active compound may be mixed under sterile conditions with a pharmaceutically acceptable carrier, and with any preservatives, buffers, or propellants which may be required.

Therapeutic/pharmaceutical compositions can be administered with medical devices known in the art. For example, in a preferred embodiment, a therapeutic/pharmaceutical composition according to the present invention can be administered with a needleless hypodermic injection device, such as the devices disclosed in US 5,399,163; US 5,383,851; US 5,312,335; US 5,064,413; US 4,941,880; US 4,790,824; or US 4,596,556. Examples of well-known implants and modules useful in the present invention include those described in: US 4,487,603, which discloses an implantable micro-infusion pump for dispensing medication at a controlled rate; US 4,486,194, which discloses a therapeutic device for administering medicaments through the skin; US 4,447,233, which discloses a medication infusion pump for delivering medication at a precise infusion rate; US 4,447,224, which discloses a variable flow implantable infusion apparatus for continuous drug delivery; US 4,439,196, which discloses an osmotic drug delivery system having multi-chamber compartments; and US 4,475,916, which discloses an osmotic drug delivery system.

Many other such implants, delivery systems, and modules are known to those skilled in the art. In certain embodiments, the compounds according to the present invention can be formulated to ensure proper distribution *in vivo.* For example, the blood-brain barrier (BBB) excludes many highly hydrophilic compounds. To ensure that the compounds according to the present invention cross the BBB (if desired), they can be formulated, for example, in liposomes. For methods of manufacturing liposomes, see, e.g., US 4,522,811; US 5,374,548; and US 5,399,331. The liposomes may comprise one or more moieties which are selectively transported into specific cells or organs, and thus enhance targeted drug delivery (see, e.g., V.V. Ranade (1989) J. Clin. Pharmacol. 29: 685). Exemplary targeting moieties include folate or biotin (see, e.g., US 5,416,016 to Low et al.); mannosides (Umezawa et al., (1988) Biochem. Biophys. Res. Commun. 153: 1038); antibodies (P.G. Bloeman et al. (1995) FEBS Lett. 357: 140; M. Owais et al. (1995) Antimicrob. Agents Chemother. 39: 180); and surfactant protein A receptor (Briscoe et al. (1995) Am. J. Physiol. 1233: 134).

In one embodiment, the compounds according to the present invention (or the compounds used in the present invention) are formulated in liposomes. In a more preferred embodiment, the liposomes include a targeting moiety. In a most preferred embodiment, the compounds in the liposomes are delivered by bolus injection to a site proximal to the desired area. Such liposome-based composition should be fluid to the extent that easy syringability exists, should be stable under the conditions of manufacture and storage and should be preserved against the contaminating action of microorganisms such as bacteria and fungi.

A "therapeutically effective dosage" for therapy/treatment can be measured by objective responses which can either be complete or partial. A complete response (CR) is defined as no clinical, radiological or other evidence of a condition, disorder or disease. A partial response (PR) results from a reduction in disease of greater than 50%. Median time to progression is a measure that characterizes the durability of the objective tumor response.

A "therapeutically effective dosage" for therapy/treatment can also be measured by its ability to stabilize the progression of a condition, disorder or disease. The ability of a compound to inhibit one or more protein kinases or to reduce the viability of cells associated with a proliferative disorder, such as cancer cells can be evaluated by using appropriate *in-vitro* assays known to the skilled practitioner, such as those described herein (in particular in the Examples below). Alternatively, the properties of a compound described in the present invention can be evaluated by examining the ability of the compound in appropriate animal model systems known to the skilled practitioner such as those described herein (in particular in the Examples below). A therapeutically effective amount of a compound according to the present invention can cure, heal, alleviate, relieve, alter, remedy, ameliorate, improve or affect the condition, disorder or disease or the symptoms of the condition, disorder or disease or the predisposition toward the condition, disorder or disease in an individual. One of ordinary skill in the art would be able to determine such amounts based on such factors as the individual's size, the severity of the individual's symptoms, and the particular composition or route of administration selected.

The pharmaceutical composition according to the invention can also, if desired, be presented in a pack, or dispenser device which can contain one or more (e.g., unit) dosage forms containing the active compound. The pack can for example comprise metal or plastic foil, such as blister pack. The pack or dispenser device can be accompanied with a leaflet or other information; in particular, that describing (either to the patient and/or the administering physician) salient information or details on the pharmaceutical composition contained in the package, such as how to administer, recommended dosages, safety and/or side-effect information.

In a particular embodiment, a pharmaceutical composition of the invention is formulated for oral administration, and in an alternative particular embodiment, a pharmaceutical composition of the invention is formulated for intravenous administration.

In one embodiment, a pharmaceutical composition of the invention is in unit dose form, and in particular may be in a unit dose form that is formulated for oral administration.

Each of such a unit dose form may comprise (e.g., it may contain) between 1 and 950mg of the compound, such as the SIK3 kinase inhibitor of the first aspect or a compound used in the fifth aspect, complex, polymorph, crystalline form, racemic mixture, diastereomer, enantiomer, tautomer, conformer, isotopically labeled form, prodrug, or combination thereof. In particular, (e.g., each of) such a unit dose form may comprise (e.g., it may contain) between 2 and 150mg of such compound; and suitably, between 10 and 150mg of such compound.

In particular of such embodiments, a pharmaceutical composition of the invention that is in unit dose form (and in particular one be in a unit dose form that is formulated for oral administration) may comprise (e.g., it may contain) - for each unit dose form - about an amount of such compound selected from the list of amounts consisting of: 2mg, 5mg, 15mg, 20mg, 50mg, 70mg, 80mg, 100mg and 140mg; in particular, comprising (e.g., containing) an amount of about 20mg, 50mg, 70mg or 100mg of a compound of the invention (or of a compound used in the invention).

In one particular embodiment, the pharmaceutical composition of the invention is (e.g., is formed as) a tablet, caplet or capsule; suitably the pharmaceutical composition of the invention (e.g., a unit dose form thereof) is a caplet. Methods to form (e.g., manufacture) tablets and caplets are, for example, described elsewhere herein.

Suitable excipients for the pharmaceutical compositions of the invention, in particular when formed as a tablet or caplet, include, and particular embodiments of such a pharmaceutical composition of the invention include those that further comprise one or more (e.g., all of) the excipients selected from the list consisting of: lactose (e.g., lactose monohydrate), microcrystalline cellulose, croscarmellose sodium, hydroxypropylcellulose and magnesium stearate.

### Therapeutic and other applications

The present application provides **a compound or composition** as specified above under the heading SIK3 inhibitors and/or TRAIL or TRAIL agonist, **or a pharmaceutical composition** as specified above under the heading "Pharmaceutical compositions" **for use as a medicament,** for example for use in therapy, a treatment, optionally in a coadministration, or combination therapy in a subject suffering from a tumour.

In one aspect the present invention pertains to SIK3 inhibitors for use in the treatment of a disease, preferably proliferative disease, characterized by a resistance to TRAIL induced cell deat (apoptosis). TRAIL resistance mechanism are known in the art, and pertain possibly to the inhibitions of intracellular apoptosis signal transduction (IAPs) and DR 4/5s due to over-expression of decoy receptor, DcR 1 and 2, and in particular, the resistance acquisition due to change of intracellular signaling system is considered to be of interest, in particular to intracellular signalling involving SIK3 (e.g. as shown in Figure 2). The major cause in change of signal transduction is known to be over-expression of antiapoptotic protein, which inhibits proapoptotic proteins functions. Therefore, the development of TRAIL sensitizer (such as a SIK3 inhibitor of the invention), which increases cancer cell line specific apoptosis by overcoming TRAIL resistance, is an essentially-required research field and provided herein. It has been reported that the TRAIL-mediated cell death pathways play an important role in the diseases including rheumatoid arthritis, diabetic renal disease, and degenerative brain disease, as well as cancer (Journal of Korean Colledge of Rheumatology Vol. 12, No.2, June, 2005; J AM Sco Nephrol 19: 904-914(2008); Cell Death Differ. 2003 Jan;10(1):134-41). A variety of approaches have thus been made to use TRAIL for relief and treatment of symptoms of autoimmune disease including arthritis, by inducing death of over-expressed immunocyte. Accordingly, TRAIL can be efficaciously used not only for treatment of the various cancers mentioned above, but also for treatment of T-cell-mediated autoimmune disease including experimental autoimmune encephalomyelitis (EAE), rheumatoid arthritis, and type I diabetes. Such diseases are included herein in certain aspects and embodiments of the invention.

Treatment including or utilising such compounds may be provided at home, the doctor's office, a clinic, a hospital's outpatient department, or a hospital. Treatment generally begins under medical supervision so that medical personnel can observe the treatment's effects closely and make any adjustments that are needed. The duration of the treatment depends on the age and condition of the patient, as well as how the patient responds to the treatment.

A person having a greater risk of developing a condition, disorder or disease may receive prophylactic treatment to inhibit or delay symptoms of the condition, disorder or disease.

The term "treatment" is known to the person of ordinary skill, and includes the application or administration of a therapeutic agent (e.g., a pharmaceutical composition containing said agent) or procedure to a patient or application or administration of a therapeutic agent (e.g., a pharmaceutical composition containing said agent) or procedure to a cell, cell culture, cell line, sample, tissue or organ isolated from a patient, who has a condition, disorder or disease, a symptom of the condition, disorder or disease or a predisposition toward a condition, disorder or disease, with the purpose to cure, heal, alleviate, relieve, alter, remedy, ameliorate, improve, affect or prevent the condition, disorder or disease, the symptoms of the condition, disorder or disease or the predisposition toward the condition, disorder or disease. Hence, the term "treatment" can include prophylactic treatment of a condition, disorder or disease, or the symptom of a condition, disorder or disease. A therapeutic agent, when used in treatment, includes the kinase inhibitors of the invention (or the compounds used in the fifth aspect of the present invention) and includes, but is not limited to, additional therapeutic agents that may be small molecules, peptides, peptidomimetics, polypeptides/proteins, antibodies, nucleotides such as DNA or RNA, cells, viruses, ribozymes, siRNA, and antisense oligonucleotides.

The disease, disorder or a condition, in the context of the herein described invention, is, in certain embodiments, a proliferative disorder (including a condition or symptom associated with such disorder).

A "proliferative disorder" refers to a disorder characterised by abnormal proliferation of cells. A proliferative disorder does not imply any limitation with respect to the rate of cell growth, but merely indicates loss of normal controls that affect growth and cell division. Thus, in some embodiments, cells of a proliferative disorder can have the same cell division rates as normal cells but do not respond to signals that limit such growth. Within the ambit of "proliferative disorder" is neoplasm or tumour, which is an abnormal growth of tissue or cells. Cancer is art understood, and includes any of various malignant neoplasms characterised by the proliferation of cells that have the capability to invade surrounding tissue and/or metastasise to new colonisation sites. Proliferative disorders include cancer, atherosclerosis, rheumatoid arthritis, idiopathic pulmonary fibrosis and cirrhosis of the liver. Non-cancerous proliferative disorders also include hyperproliferation of cells in the skin such as psoriasis and its varied clinical forms, Reiter's syndrome, pityriasis rubra pilaris, and hyperproliferative variants of disorders of keratinisation (e.g., actinic keratosis, senile keratosis), scleroderma, and the like.

A preferred tumour is a solid tumour. In another particular embodiment the proliferative disorder is (eg, the subject suffers from, or is suspected of suffering from) a solid tumour being one of those described elsewhere herein, such as pancreatic cancer, breast cancer, lung, prostate, melanoma, ovarian cancer, oesophageal cancer, sarcoma and colorectal cancer. In a certain of such embodiments, the proliferative disorder is (eg, the subject suffers from. or is suspected of suffering from) pancreatic cancer; in another of such embodiments, the proliferative disorder is (eg, the subject suffers from, or is suspected of suffering from) prostate cancer; and in yet another of such embodiments, the proliferative disorder is (eg, the subject suffers from, or is suspected of suffering from) lung cancer (eg, non-small cell lung cancer). Most preferred in context of the invention is the treatment of a tumour selected from breast cancer and melanoma.

In accordance with the present invention, a proliferative disorder, or tumour, treatable with the compounds/compositions of the invention is preferably a tumour by repolarization of the tumour microenvironment, such as by reducing regulatory T cells (Treg) and immunosuppressive myeloid cells and activating the cytotoxic T lymphocyte (CTL) activity together with promotion of the CTL to Treg ratio in the tumour.

In view of the above, it will be appreciated that the present invention also relates to the following itemised embodiments:
Item 1: A SIK3 inhibitor for use in a treatment of a proliferative disorder in a subject, wherein the treatment involves: (i) inhibiting SIK3; and thereby sensitising or re-sensitizing cells involved with the proliferative disorder to TRAIL mediated apoptosis and/or a cell-mediated immune response involving TRAIL.
Item 2: A SIK3 inhibitor for use in a treatment of a proliferative disorder in a subject, the treatment comprising exposing cells involved with the proliferative disorder in the subject to: (i) TRAIL and/or an agonist of a TRAIL receptor (DR4 or DR5) signalling; and (ii) the SIK3 inhibitor
   wherein: (i) TRAIL and/or an agonist of a TRAIL receptor (DR4 or DR5) signalling is administered to the subject; (ii) an agent that is capable of inducing or induces the exposure of the cells involved with the proliferative disorder to TRAIL and/or an agonist of a TRAIL receptor (DR4 or DR5) signalling, is administered to the subject; or (iii) the exposure of the cells involved with the proliferative disorder to TRAIL is induced by a pharmaceutical, therapeutic or other procedure that increases the amount of TRAIL in the plasma of the subject and/or in the environment of such cells; optionally,
   wherein the treatment comprises that the SIK3 inhibitor is administered to the subject in a therapeutically effective amount effective to inhibit SIK3, in particular SIK3 in the cells involved with the proliferative disorder.
Item 3: The SIK3 inhibitor for use of item 2, wherein the treatment comprises that TRAIL and/or an agonist of a TRAIL receptor (DR4 or DR5) signalling is administered to the subject; or wherein the treatment comprises that an agent that is capable of inducing or induces the exposure of the cells involved with the proliferative disorder to TRAIL and/or an agonist of a TRAIL receptor (DR4 or DR5) signalling, is administered to the subject.
Item 4: The SIK3 inhibitor for use of item 2 or 3, wherein the agent that is administered is an immune cell, such as a lymphoid cell, for example a T cell or a natural killer (NK) cell; optionally, wherein the immune cell is administered via adoptive cell transfer (ACT).
Item 5: The SIK3 inhibitor for use of item 2 or 3, wherein the exposure of the cells involved with the proliferative disorder to TRAIL is induced by a pharmaceutical, therapeutic or other procedure that increases the amount of TRAIL in the plasma of the subject and/or in the environment of such cells.
Item 6: The SIK3 inhibitor for use of any one of items 1 to 5, wherein the proliferative disorder is characterised by a resistance to TRAIL, preferably TRAIL induced apoptosis, and wherein the treatment involves inhibiting SIK3 in cells involved with the proliferative disorder by administration of the SIK inhibitor and thereby sensitizing the cells involved with the proliferative disorder to TRAIL or TRAIL receptor signalling.
Item 7: The SIK3 inhibitor for use of any one of items 2 to 6, wherein exposure of the cells involved with the proliferative disorder to TRAIL is induced by administration of a cancer immunotherapy; optionally,
   wherein such induced exposure to TRAIL is brought about by an anti-tumour vaccine; or wherein such induced exposure to TRAIL is brought about by administration of a ligand such as an antibody, preferably a monoclonal antibody; and optionally a ligand that binds to the surface of the cell(s) involved with the proliferative disorder.
Item 8: The SIK3 inhibitor for use of any one of items 2 to 7, wherein such induced exposure to TRAIL is brought about by administration of a ligand that binds to an immune checkpoint molecule.
Item 9: A substance or composition for use in a treatment of a cancer or tumour in a subject, the treatment comprising exposing cells involved with the proliferative disorder in the subject to: (i) TRAIL and/or an agonist of a TRAIL receptor (DR4 or DR5) signalling; and (ii) a SIK3 inhibitor; wherein exposure of the cells involved with the proliferative disorder to TRAIL is induced by administration of the ligand,
   wherein the substance is a ligand that binds to an immune checkpoint molecule, or wherein the composition comprises a ligand that binds to an immune checkpoint molecule.
Item 10:The SIK3 inhibitor for use of item 8, or the substance or composition for use of item 9, wherein, the immune checkpoint molecule is one selected from the group consisting of: A2AR, B7-H3, B7-H4, CTLA-4, IDO, KIR, LAG3, PD-1 (or one of its ligands PD-L1 and PD-L2), TIM-3 (or its ligand galectin-9), TIGIT and VISTA; in particular selected from: CTLA-4, PD-1 and PD-L1; optionally,
   wherein, the ligand is an antibody selected from the group consisting of: ipilimumab, nivolumab, pembrolizumab, BGB-A317, atezolizumab, avelumab and durvaluma; in particular an antibody selected from the group consisting of: ipilimumab (YERVOY), nivolumab (OPDIVO), pembrolizumab (KEYTRUDA) and atezolizumab (TECENTRIQ); or wherein, the ligand is a small molecule, such as BMS-202 or BMS-8, BMS-1001 or BMS-1166, CA-170 or CA-327. Item 11:The SIK3 inhibitor for use of any one of items 1 to 8 and 10, or the substance or composition for use of item 9, wherein the proliferative disorder is a tumour, in particular a solid tumour; optionally,
   wherein the subject is distinguished as having been previously treated with an immunotherapy and whose tumour has progressed, in particular whose tumour relapsed, recurred or did not respond; and/or
   wherein the subject is distinguished as having a tumour that has progressed after prior radiotherapy, in having a tumour that particular relapsed, recurred or did not respond to, prior radiotherapy.
Item 12:The SIK3 inhibitor for use for use of any one of the preceding items, wherein the tumour disease is a solid tumour.
Item 13:The SIK3 inhibitor for use for use of any one of the preceding items, wherein the is tumour is a recurrent and/or metastatic tumour.
Item 14:The compound or composition for use of any one of items 1 to 3 wherein the treatment is for increasing the sensitivity of the subject to TRAIL mediated apoptosis, or to apoptosis induced by an agonist of TRAIL receptor signalling (DR4 or DR5).
Item 15:The SIK3 inhibitor for use for use of any one of the preceding items, wherein the SIK3 inhibitor is a compound selected from a compound selected from the group consisting of a kinase inhibitor of the formula: 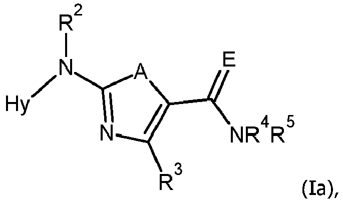 and solvates, salts, N-oxides, complexes, polymorphs, crystalline forms, racemic mixtures, diastereomers, enantiomers, tautomers, conformers, isotopically labeled forms, prodrugs, and combinations thereof; wherein Hy, R2, R3, R4, R5, A, and E re as in WO 2021/214117.
Item 16:The compound or composition for use of item 8, wherein the SIK3 inhibitor is a compound having the the formula: 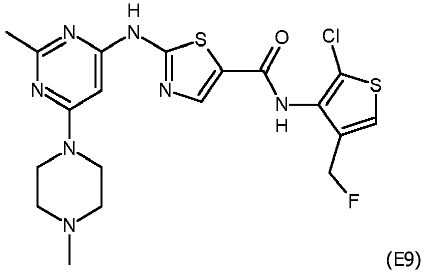 (*N*-(2-chloro-4-(fluoromethyl)thiophen-3-yl)-2-((2-methyl-6-(4-methylpiperazin-1-yl)pyrimidin-4-yl)amino)thiazole-5-carboxamide),
   and solvates, salts, N-oxides, complexes, polymorphs, crystalline forms, racemic mixtures, diastereomers, enantiomers, tautomers, conformers, isotopically labeled forms, prodrugs, and combinations thereof.
Item 17:The SIK3 inhibitor for use for use of any one of the preceding items, which is in the form of a preparation for simultaneous, separate or sequential use in therapy in the subject.
Item 18:The SIK3 inhibitor for use for use of any one of the preceding items, wherein the preparation comprises TRAIL, a TRAIL variant, a TRAIL receptor (DR4 or DR5) agonist and the SIK3 inhibitor.
Item 19**:A pharmaceutical composition** comprising TRAIL or an agonist of TRAIL signalling and an inhibitor of Salt Inducible Kinase 3 (SIK3 inhibitor), together with a pharmaceutical acceptable carrier and/or excipient.

### EXAMPLES

The examples show:

### Example 1: SIK3 inhibition increases TNF and TRAIL induced cleavage of caspase-3/7, cell surface phosphatidylserine expression and cell membrane disrupture of tumor cells

A potent inhibitor of SIK3 (compound E9) enhances death receptor mediated apoptosis in tumor cell lines of various indications including a mouse model of colorectal cancer (Figure 3A), breast cancer (Figure 3B) and melanoma (Figure 3B). Figure 3 shows a potent enhancement of death receptor induced apoptosis in various cancer cell models using three independent assays including caspase 3/7 cleavage, annexin V increase and cyctox red increase. The effect is shown for TRAIL and a TRAIL receptor agonist (anti-DR5 antibody).

## Claims

1. A SIK3 inhibitor for use in a treatment of a proliferative disorder in a subject, wherein the treatment involves: (i) inhibiting SIK3; and thereby sensitising or re-sensitizing cells involved with the proliferative disorder to TRAIL mediated apoptosis and/or a cell-mediated immune response involving TRAIL.

2. A SIK3 inhibitor for use in a treatment of a proliferative disorder in a subject, the treatment comprising exposing cells involved with the proliferative disorder in the subject to: (i) TRAIL and/or an agonist of a TRAIL receptor (DR4 or DR5) signalling; and (ii) the SIK3 inhibitor
wherein: (i) TRAIL and/or an agonist of a TRAIL receptor (DR4 or DR5) signalling is administered to the subject; (ii) an agent that is capable of inducing or induces the exposure of the cells involved with the proliferative disorder to TRAIL and/or an agonist of a TRAIL receptor (DR4 or DR5) signalling, is administered to the subject; or (iii) the exposure of the cells involved with the proliferative disorder to TRAIL is induced by a pharmaceutical, therapeutic or other procedure that increases the amount of TRAIL in the plasma of the subject and/or in the environment of such cells; optionally,
wherein the treatment comprises that the SIK3 inhibitor is administered to the subject in a therapeutically effective amount effective to inhibit SIK3, in particular SIK3 in the cells involved with the proliferative disorder.

3. The SIK3 inhibitor for use of claim 2, wherein the treatment comprises that TRAIL and/or an agonist of a TRAIL receptor (DR4 or DR5) signalling is administered to the subject; or wherein the treatment comprises that an agent that is capable of inducing or induces the exposure of the cells involved with the proliferative disorder to TRAIL and/or an agonist of a TRAIL receptor (DR4 or DR5) signalling, is administered to the subject.

4. The SIK3 inhibitor for use of claim 2 or 3, wherein the agent that is administered is an immune cell, such as a lymphoid cell, for example a T cell or a natural killer (NK) cell; optionally, wherein the immune cell is administered via adoptive cell transfer (ACT).

5. The SIK3 inhibitor for use of claim 2 or 3, wherein the exposure of the cells involved with the proliferative disorder to TRAIL is induced by a pharmaceutical, therapeutic or other procedure that increases the amount of TRAIL in the plasma of the subject and/or in the environment of such cells.

6. The SIK3 inhibitor for use of any one of claims 1 to 5, wherein the proliferative disorder is **characterised by** a resistance to TRAIL, preferably TRAIL induced apoptosis, and wherein the treatment involves inhibiting SIK3 in cells involved with the proliferative disorder by administration of the SIK inhibitor and thereby sensitizing the cells involved with the proliferative disorder to TRAIL or TRAIL receptor signalling.

7. The SIK3 inhibitor for use of any one of claims 2 to 6, wherein exposure of the cells involved with the proliferative disorder to TRAIL is induced by administration of a cancer immunotherapy; optionally,
wherein such induced exposure to TRAIL is brought about by an anti-tumour vaccine; or
wherein such induced exposure to TRAIL is brought about by administration of a ligand such as an antibody, preferably a monoclonal antibody; and optionally a ligand that binds to the surface of the cell(s) involved with the proliferative disorder.

8. The SIK3 inhibitor for use of any one of claims 2 to 7, wherein such induced exposure to TRAIL is brought about by administration of a ligand that binds to an immune checkpoint molecule.

9. A substance or composition for use in a treatment of a cancer or tumour in a subject, the treatment comprising exposing cells involved with the proliferative disorder in the subject to: (i) TRAIL and/or an agonist of a TRAIL receptor (DR4 or DR5) signalling; and (ii) a SIK3 inhibitor; wherein exposure of the cells involved with the proliferative disorder to TRAIL is induced by administration of the ligand,
wherein the substance is a ligand that binds to an immune checkpoint molecule, or wherein the composition comprises a ligand that binds to an immune checkpoint molecule.

10. The SIK3 inhibitor for use of claim 8, or the substance or composition for use of claim 9, wherein, the immune checkpoint molecule is one selected from the group consisting of: A2AR, B7-H3, B7-H4, CTLA-4, IDO, KIR, LAG3, PD-1 (or one of its ligands PD-L1 and PD-L2), TIM-3 (or its ligand galectin-9), TIGIT and VISTA; in particular selected from: CTLA-4, PD-1 and PD-L1; optionally,
wherein, the ligand is an antibody selected from the group consisting of: ipilimumab, nivolumab, pembrolizumab, BGB-A317, atezolizumab, avelumab and durvaluma; in particular an antibody selected from the group consisting of: ipilimumab (YERVOY), nivolumab (OPDIVO), pembrolizumab (KEYTRUDA) and atezolizumab (TECENTRIQ); or wherein, the ligand is a small molecule, such as BMS-202 or BMS-8, BMS-1001 or BMS-1166, CA-170 or CA-327.

11. The SIK3 inhibitor for use of any one of claims 1 to 8 and 10, or the substance or composition for use of claim 9, wherein the proliferative disorder is a tumour, in particular a solid tumour; optionally,
wherein the subject is distinguished as having been previously treated with an immunotherapy and whose tumour has progressed, in particular whose tumour relapsed, recurred or did not respond; and/or
wherein the subject is distinguished as having a tumour that has progressed after prior radiotherapy, in having a tumour that particular relapsed, recurred or did not respond to, prior radiotherapy.

12. The SIK3 inhibitor for use for use of any one of the preceding claims, wherein the tumour disease is a solid tumour, and/or is a recurrent and/or metastatic tumour.

13. The compound or composition for use of any one of claims 1 to 3 wherein the treatment is for increasing the sensitivity of the subject to TRAIL mediated apoptosis, or to apoptosis induced by an agonist of TRAIL receptor signalling (DR4 or DR5).

14. The compound or composition for use of claim 8, wherein the SIK3 inhibitor is a compound having the formula: 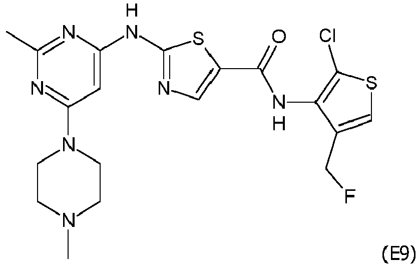 (*N*-(2-chloro-4-(fluoromethyl)thiophen-3-yl)-2-((2-methyl-6-(4-methylpiperazin-1-yl)pyrimidin-4-yl)amino)thiazole-5-carboxamide),
and solvates, salts, N-oxides, complexes, polymorphs, crystalline forms, racemic mixtures, diastereomers, enantiomers, tautomers, conformers, isotopically labeled forms, prodrugs, and combinations thereof.

15. A **pharmaceutical composition** comprising TRAIL or an agonist of TRAIL signalling and an inhibitor of Salt Inducible Kinase 3 (SIK3 inhibitor), together with a pharmaceutical acceptable carrier and/or excipient.
